(19) Europäisches Patentamt
European Patent Office
Office européen des brevets

(11) EP 0 652 764 B1

(12) EUROPEAN PATENT SPECIFICATION

(45) Date of publication and mention of the grant of the patent:
**24.05.2000 Bulletin 2000/21**

(21) Application number: **93905870.7**

(22) Date of filing: **10.02.1993**

(51) Int. Cl.[7]: **A61K 38/02**, A61K 38/04, A61K 38/08, A61K 39/12, C07K 7/06, C07K 7/08, C07K 14/00

(86) International application number:
**PCT/US93/01207**

(87) International publication number:
**WO 93/15750 (19.08.1993 Gazette 1993/20)**

(54) **USE OF SYNTHETIC PEPTIDES TO INDUCE TOLERANCE TO PATHOGENIC T AND B CELL EPITOPES OF AUTOANTIGENS**

VERWENDUNG SYNTHETISCHER PEPTIDE ZUR INDUKTION DER TOLERANZ GEGEN T- UND B-ZELL EPITOPE VON AUTOANTIGENEN

UTILISATION DE PEPTIDES SYNTHETIQUES POUR L'INDUCTION DE TOLERANCE A DES EPITOPES DE CELLULES T ET B D'AUTOANTIGENES

(84) Designated Contracting States:
**AT BE CH DE DK ES FR GB GR IE IT LI LU MC NL PT SE**

(30) Priority: **10.02.1992 US 833429**

(43) Date of publication of application:
**17.05.1995 Bulletin 1995/20**

(73) Proprietor: **DUKE UNIVERSITY**
**Durham, North Carolina 27708-0083 (US)**

(72) Inventor: **HAYNES, Barton, F.**
**Durham NC 27707 (US)**

(74) Representative:
**Peaucelle, Chantal et al**
**Cabinet Armengaud Aine**
**3, avenue Bugeaud**
**75116 Paris (FR)**

(56) References cited:
**EP-A- 0 273 716**
**WO-A-88/05783**
**WO-A-91/04273**

- **KEYSTONE SYMPOSIUM ON PREVENTION AND TREATMENT OF AIDS, KEYSTONE, COLORADO, USA, MARCH 27-APRIL 3, 1992. J CELL BIOCHEM SUPPL 0 (16 PART E). 1992. 60. CODEN: JCBSD7, COTSAMIRE D L ET AL 'SYNTHESIS OF HIV GP41 FUSION SEQUENCE AMINO-TERMINAL TO THE HYBRID T1-SP10 PEPTIDE YIELDS PEPTIDES THAT FORM HIGH MOLECULAR WEIGHT COMPLEXES IN AQUEOUS SOLUTIONS.'**
- **KEYSTONE SYMPOSIUM ON PREVENTION AND TREATMENT OF AIDS, KEYSTONE, COLORADO, USA, MARCH 27-APRIL 3, 1992. J CELL BIOCHEM SUPPL 0 (16 PART E). 1992. 10. CODEN: JCBSD7, HAYNES B F ET AL 'HIV SYNTHETIC PEPTIDES CONTAINING T AND B CELL EPITOPES AS VACCINE CANDIDATES.'**
- **Proceedings of the National Academy of Sciences, Vol. 88, issued November 1991, HART et al., "Priming of Anti-Human Immunodeficiency Virus (HIV) CD8+ Cytotoxic T Cells In Vivo by Carrier-Free HIV Synthetic Peptides", pages 9448-9452, see entire document.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 652 764 B1

- **Biochemistry Journal, Vol. 269, issued 1990, PAPADOULI et al., "Antigenic Role of Single Residues within the Main Immunogenic Region of the Nicotinic Acetylcholine Receptor", pages 239-245, see entire document.**
- **Journal of Experimental Medicine, Vol. 167, issued March 1988, GOLDING et al., "Identification of Homologous Regions in Human Immunodeficiency Virus I gp41 and Human MNC Class II B 1 Domain", pages 914-923, see entire document.**
- **Biochemical and Biophysical Research Communications, Vol. 171, No. 1, issued 31 August 1990, MURAKAMI et al., "Identification of Immunogenic Regions in Human Thyrotropin Receptor for Immunoglobulin G of Patients with Graves Disease", pages 512-518, see entire document.**

## Description

## BACKGROUND OF THE INVENTION

Field of the Invention

The present invention relates, in general, to the use of synthetic peptides to induce tolerance to immunogenic peptides. In particular, the present invention relates to a method of inducing tolerance in a mammal to an immunogenic peptide or protein comprising administering to a mammal a synthetic toleragen comprising a 2 to 20 amino acid hydrophobic peptide linked to the N-terminus or C-terminus of the immunogenic peptide or protein, under conditions such that the tolerance is induced.

Background Information

Many autoimmune diseases in animals and man are characterized by T and B cell responses to pathogenic epitopes on self antigens (Immunotherapy of Diabetes and Selected Autoimmune Diseases, G.S. Eisenbarth (ED) CRC Press, Boca Raton, 1989; Current Therapy in Allergy, Immunology, and Rheumatology-3. L.M. Lichtenstein, et al, B.C. Decker, Inc., Toronto, 1988). Examples of autoimmune diseases or disease models that are caused by autoreactive B cells responses are listed in Table 1. Examples of autoimmune diseases or disease models that are caused by autoreactive T cell responses are listed in Table 2. A method to tolerize human lymphocytes to not respond to pathogenic T and B cell epitopes of autoantigens that otherwise induce immune responses that cause tissue damage would represent a significant advance in the therapy of autoimmune diseases. Similarly, multiple clinical situations exist outside the setting of autoimmune disease, in which B and T cell responses are harmful and would advantageously be shut off or decreased. Examples of pathogenic non-autoimmune antibody responses are antibody responses to ABO incompatible erythrocytes. A method to induce tolerance against this type of immunogen would be a powerful tool for treatment of a number of similar conditions.

Recently, it has also become clear that tissue destruction in certain infectious diseases is caused by immune responses against normal tissue that are induced by infectious agents. For example, in HTLV-I infection, the clinical syndrome of HTLV-1 associated myelopathy (HAM) has been shown to be associated with the induction of cytotoxic T lymphocytes reactive with a specific region (SP4A1) OF HTLV-1 gp46 envelope glycoprotein (S. Jacobson, et al, J. Immunol. 146:1155-1162, 1991). Similarly, lymphocytic pneumonitis in HIV infection has been shown to be associated with the presence in lung lymphocytes of CTL specific for HIV infected cells (AIDS, B.D. Walker, et al, 4:177, 1990). In both HIV and HTLV-1 infection, it is thought that certain manifestations of the disease are caused by the induction of anti-viral immune responses that cross-react with normal human host antigens (G.W. Hoffman, et al, Proc. Natl. Acad. Sci. USA 88:3060-3064, 1991; H. Wigzell, et al, FASEB J. p.2406-2410, 1991; H. Golding, et al, J. Clin. Invest. 83:1430-1435).

Robinson et al have demonstrated that antibody responses to HIV envelope gp41 epitopes enhance HIV infectivity (W.E. Robinson, et al, Proc. Natl. Acad. Sci. USA, 86:4710, 1989). Recently, evidence has been presented that many if not all of the manifestations of AIDS may be caused by an autoimmune response to HLA antigens that are induced by HIV viral proteins that share sequence homologies with normal host HLA molecules (G.W. Hoffman, et al, Prac. Natl. Acad. Sci. USA 88:3060-3064, 1991; H. Wigzell, et al, FASEB J. p.2406-2410, 1991; H. Golding, et al, J. Clin. Invest. 83:1430-1435). Thus, a method of induction of tolerance (non-responsiveness) to pathogenic HIV or HTLV-1 protein epitopes (or to epitopes of any other infectious agent that induces autoreactive immune responses), would be an important and novel mode of preventing infectious tissue damage.

The ability to induce tolerance to an immune response induced by an infectious agent to prevent tissue destruction has been proposed as a method of treatment of Herpes simplex virus (HSV-1) corneal inflammation (R.L. Hendricks, et al, J. Immunol. 142:263-269, 1989).

The form of antigen has been suggested to be important regarding determination of whether a protein antigen is an immunogen or a toleragen (Reviewed in Weigle (1989) The role of the physical state of human gamma globulin in the in vivo and in vitro induction of immunological tolerance. Chapter 5G, Vol. II, p 51-57). Whereas high molecular weight aggregated gamma globulin is a potent immunogen, low molecular weight globulin is a toleragen (W.O. Weigle, Chpt. 5G, Vol. II, p.51-57, 1989). In this case, the ability of aggregated gamma globulin to induce endogenous IL1 has been suggested as the mode of immunogenicity of aggregated gamma globulin (L.C. Gahring, et al, J. Immunol. 145:1318-1323, 1990)

Others have suggested that some T cell epitopes are inherently immunogenic and some are toleragenic (D.R. Milich, et al, J. Immunol. 143:3148-3156, 1989). Milich has converted toleragenic epitopes of Hepatitis B core antigen to immunogenic epitopes by single amino acid substitutions in the T cell epitopes (D.R. Milich, et al, J. Immunol. 143:3148-3156, 1989). Benacerraf has suggested that freely diffusible antigens are toleragens whereas particulate antigens that are concentrated in cells of the reticuloendothelial system are immunogenic (Benacerraf, B. Properties of antigens in relation to responsiveness and non-responsiveness, in Immunological Tolerance, M. Landy, W. Braun, Eds. Academic Press, NY, NY 1969). In contrast, Nossal reported that the particulate polymeric antigen flagellin was a potent toleragen, and induced tolerance to antibody responses to the Salmonella flagella when injected into neonatal rats (Nossal, G Antigen Dosage in Relation to Responsiveness and Non-responsiveness, in Immunological Tolerance, M. Landy, W. Braun, Eds. Academic Press, NY, NY 1969). Finally, immunogenicity versus toleragenicity of antigens has been proposed to be due to their affinity of binding to MHC and TCR molecules (rev, in Spent et al, Science 248:1357-2363, 1990).

The present invention provides a method of modification of peptide immunogens whereby the modification changes a potent immunogen into a potent toleragen. The invention is based on the unexpected observation that the F-domain of HIV-1 gp41 confers to an antigen the ability to be a toleragen. Specifically, the hydrophobic N-terminal 12 amino acids of the gp41 envelope protein that mediate fusion of HIV to uninfected cells, the fusogenic (F) domain (M.L. Bosch, et al, Science, 244:694-697, 1989), were added C-terminal to the highly immunogenic T1-SP10 and T1-SP10(A) peptides (Table 3) (T.J. Palker, et al, J. Immunol. 142:3612-3619; M.K. Hart, et al, J. Immunol. 145:2677-2685, 1990; M.K. Hart, et al, Proc. Natl. Acad. Sci. USA 88:9448-9452, 1991). When used as an immunogen in chimpanzees, the T1-SP10IIIB and the T1-SP10IIIB(A) peptides were potent immunogens, whereas the F-T1-SP10IIIB(A) peptide (M.K. Hart, et al, Proc. Natl. Acad. Sci. USA 88:9448-9452, 1991) were not as immunogenic at either low (.1mg/kg) or at high (.5 mg/kg) doses. Moreover, challenge of the animals with the highly immunogenic T1-SP10IIIB(A) peptide at month 16 of the immunization schedule proved that the F-T1-SP10IIIB(A) immunized animals were tolerant to the T1-SP10IIIB(A) HIV gp120 env determinants.

## SUMMARY OF THE INVENTION

It is a general object of this invention to provide a method of inducing tolerance in a mammal to an immunogenic peptide or protein.

It is a specific object of this invention to provide a method of inducing tolerance in a mammal to an immunogenic peptide or protein comprising administering to the mammal a synthetic toleragen comprising a hydrophobic peptide linked to the N-terminus or C-terminus of the immunogenic peptide or protein, under conditions such that the tolerance is induced.

Further objects and advantages of the present invention will be clear from the description that follows.

## BRIEF DESCRIPTION OF THE DRAWINGS

Figure 1. Antibody Titers in ELISA Assay Against Immunizing Peptide Over Time In Chimpanzees Immunized with HIV Env Synthetic Peptides.

Figure 2. Peripheral Blood Mononuclear Cell Proliferative Responses to the T1-SP10IIIB(A) Peptide in 7 Day Tritiated Thymidine Incorporation Assays.

Figure 3. PBMC Proliferative Responses of Chimpanzees Immunized with T1-SP10 Peptides and F-T1-SP10 Peptides to PHA.

Figure 4. Elution Profile of SP10MN(A) Over a G-75 Sephadex Column.

Figure 5. Elution of T1-SP10MN(A) Over a G-75 Sephadex Column.

Figure 6. Elution of F-T1-SP10MN(A) Over a Sephadex G-75 Column.

Figure 7. Elution of F-SP10MN(A) Over G-75 Column.

Figure 8. Results of DSP Cross-linking Analysis Using F-T1-SP10IIIB(A) Peptide.

Figure 9. Hypothetical Model of F-T1-SP10IIIB(A) in Aqueous Solution.

Figure 10. Variants of T1-SP10 peptides derived from HIV MN and IIIB Envelope Sequences.

Figure 11. Time course of PBMC 3H-thymidine incorporation responses to HIV Th-B peptide, T1-SP10IIIB(A), in chimpanzees immunized with HIV envelope synthetic peptides. Animals 884 (Figure 1A) and 1028 (Figure 1B) received the Th-B peptide, T1-SP10IIIB, initially (months 1-5), then the Th-B peptide, T-SP10IIIB(A) (month 6-8). After a boost with the Th-B peptide T-SP10IIIB(A) at month 14, both animals 884 and 1028 were immunized with the HIVMN Th-B peptide, T-SP10MN(A). Panels C and D show the responses of animal 1045 (Panel C) and 1070 (Panel D) to the HIVIIIB F-Th-B peptide (month 1-14), HIVIIIB Th-B peptide (month 16) and HIVMN Th-B peptide (months 17-19). All immunizations were with the indicated peptide in IFA, except all immunizations for animal 1028 after month 4, which were with peptides in PBS alone. Solid lines show data for peak proliferative reponses (Δcpm) to a wide dose range of HIVIIIB Th-B peptide. Dotted lines indicate peak proliferative response (Δcpm) to a wide dose range of the HIVMN Th-B peptide.

Figure 12. Time course of PBMC 3H-thymidine incorporation response to PHA in chimpanzees immunized with HIV envelope synthetic peptides. Immunizations and chimpanzees as in Figure 11.

Figure 13. Time course of PBMC 3H-thymidine incorporation response to candida antigen in chimpanzees immunized with HIV envelope synthetic peptides. Immunizations and chimpanzees as in Figure 11.

Figure 14. Time course of absolute numbers of lymphocytes and lymphocytic subsets in chimpanzees immunized with HIV envelope synthetic peptides. Immunizations and chimpanzees as in Figure 11. Points represent cell number/mm3 of peripheral blood lymphocytes and lymphocyte subsets. The elevated cell numbers in animal 1028 at month 4 coincided with an abscess at the injection sites.

Figure 15. HIV envelope hybrid synthetic peptides induced anti-HIV neutralizing antibodies in goats. Goat 102A was immunized with 3 mg of the F-Th-B peptide, F-T1-SP10IIIB(A) and goat 104A was immunized with the HIVIIIB Th-B peptide, T1-SP10IIIB. Immunizations were in CFA (first dose) and IFA (doses 2-4). Neutralizing titers are titers at which reverse transcriptase production was inhibited by 90% or greater.

## DETAILED DESCRIPTION OF THE INVENTION

The present invention relates to a procedure whereby protein immunogens are derivatized by either synthesizing a hydrophobic amino acid sequence of 2 to 20 amino acids in length, N-terminal to the immunogenic protein or protein fragment, or covalently linking a hydrophobic peptide fragment of 2 to 20 amino acids in length N-terminal to the immunogenic protein or protein fragment, to yield an immunogen capable of inducing tolerance to the protein immunogen when administered to a mammal such as a primate (more preferably, humans).

In a preferred embodiment, the hydrophobic peptide is 5 to 15 amino acids in length. In yet another preferred embodiment, the hydrophobic peptide is 7 to 13 amino acids in length. In a further embodiment, the length of the hydrophobic peptide is 7, 8, 9, 10, 11, 12, or 13 amino acids in length. In yet another embodiment, the hydrophobic peptide is at least 5 amino acids in length (more preferably, at least 10 amino acids in length).

Alternatively, immunogenic proteins known to be the targets of autoantibody or auto-T cell responses can be constructed using recombinant DNA technology to form new toleragens containing hydrophobic N-terminal regions as described above. While an advantageous construction of the invention is for the hydrophobic sequence to be N-terminal to the B or T cell epitope, in certain circumstances it may be advantageous to have the hydrophobic sequence C-terminal to the B or T cell epitope.

The hydrophobic region can be a fusion protein from HIV or HIV-related viruses (see Table 5), or can be a hydrophobic sequence of amino acids that is either randomly selected or is from a non-HIV related protein.

An example of this invention for inducing tolerance to antibodies against autoantigens is for the treatment of myasthenia gravis, whereby the F-sequence is synthesized N-terminal to the main immunogenic region of the acetylcholine receptor, **WNPADYGGIK** or **WNPDDYGGVK** (I. Papdouli, et al, Biochem. J. 269:239-245, 1990). The resulting immunogen is AVGIGALFLGFL**WNPADYGGIK** or AVGIGALFLGFL**WNPDDYGGVK**.

Another example of a B cell toleragen is a hybrid protein comprising the HIV fusion domain synthesized either linearly N-terminal to B cell peptide epitopes of the insulin molecule or covalently linked to the whole insulin molecule or covalently linked or constructed using recombinant DNA techniques to a peptide insulin fragment or to the whole insulin molecule. The resulting immunogen is AVGIGALFLGFL-**insulin** or AVGIGALFLGFL-**insulin peptide fragment**. These types of toleragens can be used to prevent the onset of juvenile diabetes mellitus, (J.P. Palmer, et al, Science 222:1337-1339, 1983; B.M. Dean, et al, Diabetologia 23:339-342, 1986) and to treat patients with insulin antibodies in the setting of insulin resulin resistance (J.D. Schnatz, Dolovich, et al, J. Allergy, 46:127-1137, 1970).

Another example of a B cell toleragen is a hybrid protein comprising the HIV fusion domain synthesized either linearly N-terminal to B cell peptide epitopes of the TSH receptor molecule or covalently linked to the whole TSH receptor molecule or covalently linked or constructed using recombinant DNA techniques to a peptide TSH receptor fragment or to the whole TSH receptor molecule. The resulting immunogen is AVGIGALFLGFL-**TSH receptor** or AVGIGALFLGFL-**TSH receptor peptide fragment**. These types of toleragens can be used to treat autoimmune thyroid disease (Graves' Disease) (T. Mori, et al, Biochem. & Biophy. Res. Comm. 178:165-172, 1991; M. Murakami, et al, Biochem. & Biophy. Res. Comm. 171:512-518, 1990). Table 6 summarizes B cell epitopes on the thyrotropin (TSH) receptor to which Graves' patient sera bind (T. Mori, et al, Biochem. & Biophy. Res. Comm. 178:165-172, 1991; M. Murakami, et al, Biochem. & Biophy. Res. Comm. 171:512-518, 1990; O. Takai, et al, Biochem. & Biophy. Res. Comm. 179:319-326, 1991; T. Piraphatdis, et al, Biochem. & Biophy. Res. Comm. 172:529-536, 1990). Of interest is the sequence YYVFFEEQEDEIIGF identified by 2 studies that inhibits the TSH activity of the autoantibodies (T. Mori, et al, Biochem. & Biophy. Res. Comm. 178:165-172, 1991; O. Takai, et al, Biochem. & Biophy. Res. Comm. 179:319-326, 1991). Thus constructs for inducing tolerance to anti-TSH antibodies in Graves' disease are
AVGIGALFLGFL**YVFFEEQEDEI** or
AVGIGALFLGFL**HQEEDFRVTCKDIQRIPSLPPSTQT** or
AVGIGALFLGFL**LRQRKSVNALNSPLHQEYEENLGDSIVGY** or

AVGIGALFLGFL**YYVFFEEQEDEIIGF** or
AVGIGALFLGFL**YKELPLLKFL**.

An example of the use of this invention in the induction of tolerance to autoimmune T cell antigens is a hybrid protein comprised of the HIV fusion domain synthesized either linearly N-terminal to T cell peptide epitopes of the myelin basic protein molecule or covalently linked or constructed using recombinant DNA techniques to a myelin protein molecule. The resulting immunogen is AVGIGALFLGFL-**myelin basic protein** or AVGIGALFLGFL-**myelin basic protein peptide fragment**. In the case of the myelin basic protein peptide fragment, the encephalitogenic T cell epitopes are known, one of which is contained in sequence 69-89 of bovine myelin basic protein (H. Offner, et al, J. Immunol. 141:3828-3832, 1988). In this case, one formulation of the toleragen is AVGIGALFLGFL**GSLPQKSQRSQDENPVVHF**. These types of toleragens can be used to treat experimental autoimmune encephalomyelitis, which is thought to be an excellent model of human multiple sclerosis (K.W. Wucherpfenning, et al, Immunol. Today, 12:277-281, 1991). When the specific epitopes are identified that are the T cell targets in multiple sclerosis, then those sequences can be substituted in the peptide above, and used to tolerize T cells to the pathogenic T cell epitope of whatever the protein antigen turns out to be involved in multiple sclerosis.

Another example of this invention for induction of tolerance to autoimmune T cell antigens is a hybrid protein comprising the HIV fusion domain synthesized either linearly N-terminal to T cell peptide epitopes of the retinal S protein molecule or covalently linked to the whole retinal S protein molecule or covalently linked or constructed using recombinant DNA techniques to retinal S antigen fragment or to the whole retinal S antigen molecule. The resulting immunogen is AVGIGALFLGFL-**retinal S protein** or AVGIGALFLGFL-**retinal S protein peptide fragment**. In the case of the retinal S protein peptide fragment, the pathogenic T cell epitopes are known, one of which is present in the sequence 1169-1191 of retinal S protein (H. Sanui, et al, Exp. Med., 169:1947-1960, 1989). In this case, one formulation of the toleragen is AVGIGALFLGFL**PTARSVGAADGSSWEGVGVV**. These types of toleragens can be used to treat experimental autoimmune retinouveitis, which is thought to be an excellent model of human inflammatory eye diseases such as Bechet's syndrome and idiopathic retinouveitis (H. Sanui, et al, Exp. Med., 169:1947-1960, 1989). When the specific epitopes are discovered that are the T cell targets in human inflammatory eye disease, then those sequences can be substituted in the peptide above, and used to tolerize T cells to the pathogenic T cell epitope of whatever the protein antigen turns out to be in human retinouveitis.

For the treatment of pathogenic immune responses induced by an infectious agent, an example of the invention is the treatment of HTLV-I associated myelopathy syndrome seen in tropical spastic paraparesis (rev, in Jacobson et al J. Immunol. 146:1155-1162, 1991). In this disease, there is strong evidence that the neurologic disease is caused by the induction of cytotoxic T cells (CTL) against HTLV-I infected cells in the central nervous system (S. Jacobson, at al, J. Immunol. 146:1155-1162, 1991). Jacobson, et al have shown that one primary region of HTLV-I env gp46 that induces CTL in tropical spastic paraparesis (TSP) is aa196-209 of gp46 as defined by peptide SP4a1 (S. Jacobson, et al, J. Immunol. 146:1155-1162, 1991; T.J. Palker, et al, J. Immunol., 142:971-978, 1989; A. Kurata, et al, J. Immunol., 143:2024-2030, 1989). Thus, to treat TSP, the present invention can be embodied by the hybrid peptide AVGIGALFLGFL**LDHILEPSIPWKSKK**. When new pathogenic CTL epitopes of HTLV-I are discovered, the therapeutic construct can be F-X where F is the hydrophobic sequence and X is the CTL epitope of the infectious agent.

The clinical manifestations of HIV have been postulated to be due to autoimmune responses induced by components of HIV that have sequence homology to human MHC Class I or Class II molecules (G.W. Hoffman, at al, Prac. Natl. Acad. Sci. USA 88:3060-3064, 1991; H. Wigzell, at al, FASEB J. p.2406-2410, 1991; H. Golding, et al, J. Clin. Invest. 83:1430-1435; F. Grassi, et al, J. Ex. Med., 174:53-62, 1991; J.A.T. Young, Nature, 333:215, 1988; H. Golding, at al, J. Exp. Med., 167:914-923, 1988). For the treatment of HIV infection, the present invention can comprise a series of hybrid peptides, each peptide containing an N-terminal hydrophobic peptide such as the HIV gp41 fusion domain (Table 5) and a C-terminal peptide from each of the regions of HIV env proteins bearing sequence homology MHC class I or class II molecules (G.W. Hoffman, at al, Prac. Natl. Acad. Sci. USA 88:3060-3064, 1991; H. Wigzell, et al, FASEB J. p.2406-2410, 1991; H. Golding, at al, J. Clin. Invest. 83:1430-1435; F. Grassi, et al, J. Ex. Med., 174:53-62, 1991; J.A.T. Young, Nature, 333:215, 1988; H. Golding, et al, J. Exp. Med., 167:914-923, 1988) (Table 7). Alternatively, it may be advantageous to treat HIV infected individuals with F-X peptides where F is a hydrophobic peptide such as the fusogenic domain of HIV and X is a peptide fragment of HIV that is immunogenic to T or B cells. In this situation, a mixture of peptides would be used to inhibit destructive anti-HIV immune responses that were damaging host HIV-infected antigen-presenting cells. Examples of this type of peptide are shown in Table 3 and Figure 10, and were the peptides used that tolerized chimpanzees in Figures 1 and 2 to both T1-SP10(A) determinants and to whole gp120 protein (Table 4).

The present invention is described in further detail in the following non-limiting Example.

Example 1

Antibody titers in ELISA assay against immunizing peptide over time in chimpanzees immunized with HIV env synthetic peptides are shown in Figure 1. For animals 884 and 1028, the peptide used in the ELISA assay was T1-SP10IIIB. For animals 1045 and 1070, the peptide used in the ELISA assay was F-T1-SP10IIIB(A). All immunizations were in incomplete Freund's Adjuvant (IFA) + PBS (1:1) except for animal 1028 that developed IM abscesses after immunization no. 3, and had one immunization held, then had all subsequent immunizations in PBS only. As can be seen, T1-SP10 peptides were excellent immunogens in animals 884 and 1028, while T1-SP10 peptides with the HIV gp41 fusion (F) domain synthesized N-terminal to the T1-SP10 peptide did not induce antibody titers as high or as of long duration as did peptides without the F domain.

It is important to note that animals 1045 and 1070 were challenged at month 16 with the immunogen T1-SP10IIIB(A) that induced such good antibody titers in animals 884 and 1028. Animals 1045 and 1028 did not respond to T1-SP10IIIB(A) in IFA, thus demonstrating that they were tolerant to the T1-SP10(A) from their prior immunizations with F-T1-SP10IIIB(A) peptide. It is also important to note that while boost of 884 at week 14 gave a rise in titer to T1-SP10IIIB(A) peptide, boost of 1028 at the same time did not. Boost of 884 was with IFA, while boost of 1028 was with no adjuvant, but rather only PBS.

Peripheral blood mononuclear cell proliferative responses to the T1-SP10IIIB(A) peptide in 7 day tritiated thymidine incorporation assays is shown in Figure 2. T1-SP10IIIB and T1-SP10IIIB(A) peptides induced high levels of proliferation of circulating PBMC in animals 884 and 1028. These levels fell to non-detectable levels after a 6 month rest (month 14) but rose again in animals 884 and 1028. Proliferative responses in animal 1028 rose with each boost after the 6 month rest even though the immunizations were in PBS alone with no adjuvant. As with B cell response, animals 1045 and 1070, immunized with F-T1-SP10IIIB(A) peptide, did not proliferate to T1-SP10IIIB(A) peptide. When these latter two animals were immunized with the T1-SP10IIIB(A) peptide that was a good immunogen in 884 and 1028, neither of the animals 1045, 1070 developed a proliferative response to T1-SP10IIIB(A)--thus proving that the addition of the F-domain N-terminal to the T1-SP10 peptide created a toleragen that tolerized animals 1045 and 1070 to the T1 and SP10 regions of gp120. As shown in Table 4, while animals 884 and 1028 both responded in proliferative assays to native gp120, animals 1045 and 1070 were tolerant to native gp120 as well as to immunizing peptides.

PBMC proliferative responses of chimpanzees immunized with T1-SP10 peptides and F-T1-SP10 peptides to PHA are shown in Figure 3. Data show that while animals 1045 and 1070 were tolerant to T1 and SP10 regions of HIV gp120, PBMC PHA responses in these animals throughout the immunization period were normal.

Similar results were obtained with peripheral blood mononuclear cell (PBMC) responses to candida antigen in 7 day <u>in vitro</u> stimulation assay (not shown). Thus, while specifically tolerant to T1 AND SP10(A), HIV env determinants, animal 1029 and 1045 were not generally immunosuppressed and could respond to candida to PHA stimulation <u>in vitro</u>.

The effect on peptide quartenary structure of placement of a hydrophobic sequence N-terminal to a T cell and/or a B cell determinant was examined. Using G-75 chromatography in aqueous buffers and crosslinking of peptide monomers using the heterobifunctional agent Dithiobis (succinimidylpropionate) (DSP), it was determined that addition of a hydrophobic sequence such as the fusion (F) domain of HIV or HIV-like retroviruses confers on the T1-SP10(A) or the SP10(A) peptide the ability to form high molecular weight aggregates, that are likely in the form of protein micelles.

An elution profile of SP10MN(A) over a G-75 Sephadex column is shown in Figure 4. 4mg of each peptide in 2ml 50mM Tris-HC1 (pH 7.5) containing 100mM KC1 and 5% glycerol, was applied directly to a 90 x 1.6 cm column of Sephadex G-75 equilibrated with 50mM Tris-HC1 (pH 7.5) containing 100mM KC1. The sizing column was calibrated with blue dextran (200,000), bovine serum albumin (66,000), bovine erythrocyte carbonic anhydrase (29,000), horse heart cytochrome C (12,400) and bovine lung aprotinin (6,500). The elution position of each peptide was determined by continuous measurement of eluent absorbance at OD 280. The corresponding molecular weight of each peptide peak was calculated from the calibration curve of the column. Each peptide was also applied to the column equilibrated with the same buffer containing 0.1% C12E9 [polyoxyethylene (9) lauryl ether]. The SP10MN(A) peptide (predicted Mr=2878) migrated as forms of 65 Da or lower. Similarly, the T1-SP10MN(A) (predicted Mr=4771) peptide also migrated as low mw forms ranging from 12,000 Da to 6,500 Da (Figure 5). In contrast, both the F-SP10MN(A) (Mr=4038) and the F-T1-SP10MN(A) peptides (Mr=5930) contained high molecular weight forms that migrated at ~66,000 Da (Figures 6 and 7). Methods used in Figures 5, 6, and 7 were as in Figure 4.

The results of DSP cross-linking analysis using F-T1-SP10IIIB(A) peptide are shown in Figure 8. Lane C shows the form of the peptide with no DSP added in PBS when run under non-reducing conditions in SDS-PAGE. Lanes D,E,F, and G show the effect on peptide MW when the peptide is cross-linked with 6.25μg (D), 12.5μg (E), 25μg (F) and 50μg (G) of DSP prior to SDS-PAGE. Lane H shows the results of addition of 2-ME to peptide cross-linked with 50μg of DSP and then run under reducing conditions in SDS-PAGE showing all of the cross-linked forms seen in lane H and all the multiple forms seen in non-reduced, non-cross-linked peptide seen in lane C, were now reduced to two bands at 7000 kDa. At present, the nature of the two bands in this peptide under reducing conditions is unknown; these two bands can be purified by cutting the bands out of preparative gels and can be analyzed by mass spectroscopy and sequenced. Lanes A and B show the results of crosslinking F-T1-SP10IIIB(A) peptide in the presence to Triton-X 100 1% and run under reducing (A) and non-reducing conditions (B). Data demonstrate that the apparent hydrophobic interactions holding the high MW complexes together are resistant to disruption by this detergent.

A hypothetical model of F-T1-SP10IIIB(A) in aqueous solution is shown in Figure 9. The model shows protein micelle formation with the hydrophobic fusion domain (F) regions of the peptide in the core of the micelle with the hydrophilic V3 regions projecting outward.

Examples 2-8

The following experimental details and protocols are referenced in Examples 2-8.

Peptides: Peptides used in Examples 2-8 that follow are listed in Table 8. Peptide synthesis was performed using either t-boc or f-moc chemistry with a peptide synthesizer (A431; Applied Biosystems, Inc. Foster City, CA). Peptides were purified using HPLC, and the molecular weight was determined by fast atom bombardment mass spectrometry (R. B. Van Breeman, North Carolina State University, Raleigh, NC) using a double-focusing mass spectrometer (HXIIOHF; Joel Ltd., Tokyo, Japan). For Th-B and F-Th-B peptides (Table 8), expected molecular mass of F-Th-B peptide, F-T1-SP10IIIB(A), was 5908, observed was 5907; expected molecular weight of Th-B peptide, T1-SP10III.B, was 4061, observed was 4062; expected and observed molecular weight of Th-B peptide, T1-SP10IIIB(a) was 4,749, and expected and observed molecular weight of Th-B peptide, T1-SP10MN(A), was 4771. For the peptides used in the following Examples (Table 8), the peptide amounts are gross weights. The % water by Karl Fisher Test (Galbraith Laboratories, Inc. Knoxville, TN) for each peptide was F-T1-SP10IIIB(A), 6%; T1-SP10IIIB(A), 8%; T1-SP10IIIB, 6% and T1-SP10MN(A), 8%.

Animals: Chimpanzees were housed at the New Mexico State University Primate Facility at Alamogordo, NM. Chimpanzee No. 884 (15 yrs. old) and 1028 (12 yrs. old) had the same sire; animal 1045 (10 yrs. old) and 1070 (11 yrs. old) were unrelated to each other and to animals 884 and 1028. Outbred goats were housed at the Duke University Animal Facilities.

Immunizations: For goats, 3 mg of peptide were injected intramuscularly in each gluteal region in complete Freund's adjuvant (CFA) (1st dose), then incomplete Freund's adjuvant (IFA) (subsequent doses). For immunization of chimpanzees, varying doses of peptides were injected IM in IFA in a total volume of 4 cc, with 1 cc injected into right and left upper arms and thighs.

ELISA Assays: 2 µg of Th-B peptide, T1-SP10IIIB, or rgp120IIIB (Repligen Corp., Cambridge, MD) in CBC buffer (15 mM $Na_2C0_3$, 35 mM $NaHCO_3$, pH9.6) was incubated overnight in each well of a 96 well flat bottom plate (Costar 3590). Wells were blocked with CBC buffer supplemented with 3% bovine serum albumin (BSA) for at least 2 hrs and then were washed 3 times with PBS, 0.05% Tween 20. Primary antibody at various concentrations in serum diluent (95 ml PBS, 0.05% Tween 20, supplemented with 5 g BSA in 2 ml normal serum from same species as secondary antibody) was incubated for 90 min at 20°C. After washing three times, alkaline phosphatase-conjugated secondary antibody was added to each well (60 min at RT) and the plates washed. Substrate (1 mg/ml p-nitrophenyl phosphate, Sigma Chemical Co., St. Louis, MO) in 0.05M CBC-0.002M $MgCl_2$ was added to each well, and plates developed (60 min, 20°C) in the dark and read at 405 nm on an ELISA reader (Anthros; Denley Instruments Co., Durham, NC). Endpoint ELISA antibody titers were defined as the serum titer at which the experimental/control (E/C) OD value ≥ 3.0.

HIV Neutralization Assays: The ability of chimpanzee or goat serum antibodies to neutralize HIV was determined in syncytium inhibition assay and reverse transcriptase inhibition assay as previously described (Palker et al, J. Immunol. 142:3612 (1989); Palker et al, Proc. Natl. Acad. Sci. USA 85:1932 (1988)). Sera were heat inactivated (30 min, 56°C) prior to each assay.

PBMC Isolation and In Vitro $^3$H-Thymidine Incorporation Assays: Chimpanzee or goat PBMC was isolated by standard density centrifugation techniques (Palker et al, J. Immunol. 142:3612 (1989); Haynes et al, Science 215:298 (1982)). In vitro assays of $^3$H-thymidine incorporation were performed as described (Palker et al, J. Immunol. 142:3612 (1989); Hart et al, J. Immunol. 145:2697 (1990)). For chimpanzee PBMC assays, in vitro cultures were performed using 10% normal chimpanzee serum. Antigens used in PBMC proliferation were the Th-B peptides, T1SP10IIIB(A) and T1-SP10MN(A), (Table 8), and Candida albicans antigen (Greer Laboratories, Inc. Lenoir, NC). PHA (Burroughs Wellcome, Research Triangle Park, NC) was used in a wide dose range as a mitogen in 3 day PBMC $^3$H-thymidine incorporation assays (Palker et al, J. Immunol. 142:3612 (1989); Hart et al, J. Immunol. 145:2697 (1990)).

$\Delta$cpm = experimental cpm - control cpm .

Immunization Schedule: Because of previous studies demonstrating the immunogenicity of Th-B peptides in goats and rhesus monkeys (Hart et al, J. Immunol. 145:2697 (1990)), the initial comparison of peptide designs when this study began in 1989 was monthly injections of Th-B versus F-Th-B peptides (Table 8) at a dose of approximately 0.1 mg/kg (6 mg/animal). When neither peptide design induced neutralizing anti-HIVIIIB antibodies, the peptide doses were increased to approximately 0.5 mg/kg (30 mg/animal) and the right-hand side neutralizing sequence of HIVIIIB gp120 V3 loop (the (A) region) (Hart et al, Proc. Natl. Acad. Sci. USA 88:9448; Rusche et al Proc. Natl. Acad. Sci. USA 85:3198)) (Table 8) was added to the Th-B peptide to enhance the ability of this peptide to induce anti-HIVIIIB neutralizing antibodies. After 3 monthly injections with either ~0.5 mg/kg (30 mg) Th-B or F-Th-B peptide, the animals were rested for 6 months, and then reimmunized with either F-Th-B or Th-B with sequences from HIVIIIB, or with the Th-B peptide containing HIV env gp120 V3 sequences from the HIVMN isolate.

Flow Cytometry: Chimpanzee PB mononuclear cells were studied by standard flow cytometry methods using a flow cytometer (751; Coulter Electronics, Inc., Hialeah, FL). PB lymphocytes were identified by the following markers; total T cells, CD3; T cell subunits, CD4 and CD8; B cells, CD19; and NK cells, CD56 and CD16.

Example 2

Immunogenicity of Th B and F Th B Peptides in Chimpanzees and Goats for Anti-Peptide and Anti-HIV gp 120 Antibody Responses

For chimpanzees immunized with HIVIIIB Th-B peptides (chimpanzee nos. 884 and 1028), antibody to immunizing peptide rose during the initial immunization period (Table 9). Chimpanzee no. 1028 developed an abscess at the immunization site, did not receive the month 5 immunization, and all subsequent immunizations after month 5 in animal 1028 were in PBS alone. Whereas peak endpoint ELISA anti-peptide antibody titer at month 4 in animal 1028 was 1:819,200, antibody titers fell in animal 1028 after IFA was deleted from the immunogen, and remained low throughout the remainder of the immunization period (Table 9). In chimpanzee no. 884, antibody titers rose at month 7 to 1:204,800 after 5 immunizations with Th-B peptides. Continued immunization of animal 884 with high doses of Th-B peptide (30 mg/dose) resulted in no further increases in antibody titer (Table 9).

In contrast, anti-peptide antibody levels were much lower during months 1-10 of immunization of animals 1045 and 1070 with HIVIIIB F-Th-B peptide, with peak antibody levels against immunizing peptide of 1:25,600 and 1:12,800 at month 7 for animals 1028 and 1070, respectively (Table 9). After a 6 month rest for all four animals, animals 884 and 1028 were immunized at month 14 with 6 mg of Th-B peptide. In chimpanzee no. 884, boosting with Th-B peptide in IFA at month 14 resulted in rise in titer of anti-peptide antibody to 1:102,400, while boosting of animal 1028 with peptide in PBS alone led to no antibody rise (Table 9).

In contrast, animals 1045 and 1070 were immunized at month 14 with 1 mg (~0.016 mg/kg) of F-Th-B to determine if the prior doses of F-Th-B peptide were excessive and induced high zone tolerance, and if smaller amounts of F-derivatized peptide would be more immunogenic. Immunization of both chimpanzee nos. 1045 and 1070 with 1 mg of F-Th-B peptide after a 6 month rest resulted in only minimal rises in serum titers of anti-peptide antibody to 1:800 (Table 9).

To determine if chimpanzees 1045 and 1070 were tolerant to Th-B peptides, both animals were immunized on month 16 with HIVIIIB Th-B peptide, T1-SP10IIIB(A). Both animals 1045 and 1070 responded minimally to boosting with Th-B peptide with an antipeptide antibody responses to 1:1600 and 1:3200, respectively, demonstrating that animals 1045 and 1070 were hyporesponsive at month 16 to Th-B HIV env epitopes (Table 9).

Example 3

Immunization of Animals 1045 and 1070 with HIVMN Th-B Peptide Induced High Levels of Antipeptide Antibodies

Using a previously described strategy of breaking B cell tolerance by immunization with an immunogen that is different from, but structurally related to, the tolerogen (Weigle, Natural and Acquired Immunologic Unresponsiveness (1967) Chapter 4, pp. 57-151), animals 1045 and 1070 were next immunized with the HIVMN Th-B peptide. The TH-B peptide from HIVMN contained the same Th (T1) gp 120 sequence as the HIVIIIB Th-B peptide, but contained different B cell gp 120 V3 B cell epitope sequences than those in the HIVIIIB Th-B peptide (Table 8). After 2 immunizations with Th-B of HIVMN, beginning at month 17, both chimpanzee nos. 1045 and 1070 had prompt rises in titer of antibodies to HIVIIIB (Table 9) and to HIVMN Th-B peptide (not shown) to antibody levels that were higher than had previously been obtained during the prior 18 months of study. At month 20, endpoint ELISA titers to the HIVMN Th-B peptide were 1:102,400 for animal 1045 and 1:204,800 for animal 1070.

Example 4

Chimpanzee B Cell Antibody Responses to Recombinant HIVIIIB gp120 During the 20-Month Immunization Course

Endpoint ELISA antibody titers against recombinant HIVIIIB gp 120 were determined for sera from months 4-7 and 16-20 to correlate peak anti-peptide antibody levels with anti-gp120 HIV envelope antibody levels. It was found that peak anti-gp120 antibody levels in chimpanzee nos. 884 and 1028 during months 4-7 were both 1:25,600, whereas peak titers to gp120 in animals 1045 and 1070 during the same period were 1:6,400 and 0, respectively. As with anti-peptide antibody levels, boosting after a 6 month rest with peptide in PBS in chimpanzee 1028 did not boost anti-gp120 antibodies.

Boosting with F-Th-B peptide at month 14 and with HIVIII TH-B at month 16 in animals 1070 and 1045 resulted in minimal rises in anti-gp120 antibody titers by month 17 (to 1:12,800). In contrast, boosting chimpanzees 1045 and 1070 with HIVMN Th-B peptide at month 17 induced high levels of anti-gp120IIIB antibody in both animals (1:102,400 and 1:51,200, respectively) by month 20 that rose coincident with rises in levels of anti-peptide antibody.

Example 5

Induction of Anti-Peptide and Anti-gp120 PBMC Proliferative Responses by HIV Env Peptides

Whereas HIVIIIB Th-B peptides induced high levels (>100,000 $\Delta$cpm/$10^6$ cells) of PBMC $^3$H-thymidine incorporation (animals 884 and 1028) (Figures 11A and 11B) during months 1-8, F-Th-B peptide did not induce levels of $^3$H-thymidine incorporation above 100,000 $\Delta$cpm/$10^6$ cells during the same period (Figures 11C and 11D). Immunization of animals 1045 and 1070 with Th-B peptide at month 16 did not induce the presence of circulating PBMC capable of proliferating to Th-B peptide <u>in vitro</u> (Figures 11C and 11D).

Interestingly, Th-B peptides at month 14-18 boosted PBMC proliferative responses in animal 1028, while anti-peptide antibody responses in animal 1028 during this time were not boosted (Figure 11B and Table 8).

Next, $^3$H-thymidine incorporation of chimpanzee PBMC to either recombinant gp120IIIB or to native gp120IIIB was tested. Table 4 shows the peak $^3$H-thymidine incorporation of chimpanzee PBMC to HIVIIIB gp120 for each animal during months 1-13, and demonstrates that neither chimpanzee no. 1070 nor 1045 (receiving F-Th-B peptide) had PBMC-proliferative responses to gp120 of greater than $E/C>2$ throughout the first 13 months of study. In contrast, animals 884 and 1028 (receiving Th-B peptides) did have anti-gp120 proliferative responses during the same period (Table 4).

To determine if PBMC proliferative responses to mitogenic or antigenic stimuli other than HIV immunogens were normal in the F-Th-B-immunized chimpanzees over the 20 months of study, we also measured PBMC proliferative responses to PHA were also measured (Figure 12) and to <u>Candida</u> (Figure 13). While peak PHA PBMC proliferative responses were nearly identical in the four chimpanzees, Candida PBMC-proliferative responses varied from animal to animal and from month to month. However, in animals 1045 and 1070, it was found that <u>Candida</u> responses were intermittently present during the time of immunization with F-Th-B peptide at levels that were similar to levels present before the immunizations were begun (Figures 13C and 13D).

Example 6

Characterization of PB Lymphocyte Subsets During Immunization of Chimpanzees With HIV Env Peptides

To determine if immunization with either HIV env peptide type had effects on the number of circulating chimpanzee T, B or NK cell populations, the absolute numbers of these cell types were determined throughout the immunization period (Figure 14, Table 10). Whereas preimmunization (before) and postimmunization (during) lymphocyte levels in animals 884 and 1028 were not significantly different (Table 10), animal 1045 became relatively lymphogenic ($p<0.001$) during the course of immunization with F-Th-B peptide with the lymphocyte count 650/mm$^3$ at week 12, compared to preimmunization levels of 2815 and 2597 lymphocytes/mm$^3$ in months 1 and 2, respectively (Figure 14C). Whereas T cell levels significantly dropped an average of 59% and 44% in chimpanzee nos. 1045 ($p>0.001$) and 1070 ($p>0.02$), respectively, during the immunization period, T cell levels did not significantly change in animals 884 and 1028 during the same time ($p>0.1$) (Table 10). B and NK cell levels dropped significantly in animal 1045, but did not change in animals 1070, 884 and 1028 (Table 10). Taken together, these data demonstrated that immunization with the F-derivatized HIV env peptide induced decreases in absolute levels of circulating T cells in both animals 1045 and 1070, and in B and NK cell levels in animal 1045, whereas immunization of chimpanzee nos. 884 and 1028 with HIV Th-B env peptides lacking the F domain did not significantly affect circulating lymphocyte levels.

Example 7

Ability of HIVIIIB F-Th-B and Th-B Peptides to Induce Anti-HIVIIIB Neutralizing Antibodies in Goats

To determine if the F-Th-B peptide used in the initial phase of the chimpanzee immunization protocol was immunogenic in another species, 3 mg of either F-Th-B or Th-B peptide were used to immunize goats three times over 2 months and then used to boost goats after an 8 month rest (Figure 15). It was found that after the fourth immunization, both peptides were capable of inducing serum anti-HIVIIIB neutralizing antibodies (Figure 15), and capable of inducing high levels ($\geq$500,000 $\Delta$cpm/$120^6$ cells) of PBMC $^3$H-thymidine incorporation <u>in vitro</u> to Th-B or F-Th-B peptides. In addition, serum endpoint ELISA titers of antibodies to immunizing peptide were the same in Th-B and F-Th-B-immunized goats. Thus, failure of the F-Th-B peptide to induce high levels of anti-peptide antibodies and PBMC-proliferative responses in chimpanzees was not due to lack of an inherent immunogenicity of the HIVIIIB F-Th-B peptide, but rather was due to a specific effect of the F-derivatized peptide in chimpanzees.

Example 8

HIVMN Th-B Env Peptide Induced Anti-HIV Neutralizing Antibody in Chimpanzees

During the first 17 months of the immunization trial, serum-neutralizing antibodies against HIVIIIB were always undetectable in syncytium inhibition assay and were ≤ 1:45 in reverse transcriptase inhibition assay. However, following immunization of animals 1045 and 1070 at month 17 with HIVMN Th-B peptide, anti-HIV neutralizing antibodies were seen in syncytium inhibition assay (Table 11).

To determine why antibodies against HIVIIIB Th-B peptides did not neutralize HIVIIIB in vitro during the first 17 months of immunization, sera from the early peak anti-HIVIIIB peptide antibody responses (month 6) were assayed for reactivity to the individual epitopes of the Th-B peptides. It was found that at the time of initial titers of anti-Th-B peptide responses, most of the antibody reactivity in sera from animals 884 and 1028 was indeed directed to the primary amino acid sequence of the neutralizing V3 loop region defined by the peptide (TRKSIRIQRGPGR) (Table 12,). These data indicate that antibodies made by chimpanzee nos. 884 and 1028 at 7 months after immunization with the HIVIIIB Th-B HIV env peptides did not recognize the appropriate secondary V3 loop structure(s) necessary for neutralizing HIVIIIB, although the animals did make antibody responses to the correct primary amino acid sequences of the neutralizing V3 B cell determinant of HIVIIIB gp120.

Example 9

Regarding the induction of tolerance, additional clinical syndromes that might be treated using Fusion domain or Fusion domain-like peptides synthesized N- or C-terminal to an otherwise immunogenic antigen is in hypersensitivity to bee or wasp venom antigens and hypersensitivity to plant or animal allergens. The nucleotide and amino acid sequences of a number of allergens have now been synthesized, and those regions of the allergen proteins that induce IgE antibodies or T helper cell responses that help to induce IgE responses are being mapped. Thus the primary structure of grass pollen (Silvanovich et al J. Biol. Chem. 266:1204-1220, 1991; Griffith et al FEBS Letters 279:210-215, 1991; Perez et al J. Biol. Chem. 265:16210-16215, 1990; Singh et al Proc. Natl. Acad. Sci. USA 88:1384-1388, 1991), mite allergens (Tovey et al J. Exp. Med. 170:1457-1462, 1989; Yasel et al J. Immunol. 148:738-745 1992; Chua et al J. Exp. Med. 167:175-182, 1988; Chua et al Int. Arch. Allergy Appl. Immunol. 91:118-123, 1990), hornet venom (Fang, et al Proc. Natl Acad. Sci., USA 85:895-899, 1988), and tree pollen (Ebner et al J. Immunology 150:1047-1054, 1993; Jarolim et al Int. Arch. Allergy Appl. Immunol. 90:54-60, 1989; Valenta et al Science 253:557-560, 1991). For some of these allergen proteins, T cell epitopes have been mapped (Ebner et al J. Immunology 150:1047-1045, 1993) while for others, likely T cell sites and hydrophilic B cell determinants can be predicted using computer algorithms (Kyte and Doolittle J. Mol. Biol. 157:105-132, 1982: Rothbard and Taylor EMBO J. 7:93, 1988; Margalit et al J. Immunol. 138:2213, 1987) and tested by synthesizing peptides and injecting animals, or by reacting patient serum antibodies or peripheral blood T cells with synthesized peptide in in vivo assays. Once indentified, T and B cell epitopes of bee, wasp or other allergens can be synthesized with a F domain or F-domain-like peptide N- or C-terminal to the allergenic T or B cell peptide, and the F-Allergen epitope hybrid peptide used to inject into patients that are sensitive to the allergen epitope. By this method, a patient can be made tolerant to the allergen epitope in the same manner as chimpanzees were made tolerant to T1-SP10 HIV env peptides by immunizing them with F-T1-SP10(A) peptide (Haynes et al J. Exp. Med. in press, 1993). Thus, in addition to treating autoimmune disease, F-derivatizing allergen T or B cell immunogenic peptides could product tolerogenic peptides for the treatment of allergic diseases.

A new technology has been developed whereby injection in vivo of cDNAs with a powerful promoter and encoding immunogenic peptides or proteins has been found to promte internalization and expression of cDNAs in host cells (Wolff et al Science 247:1465, 1990). Thus, the above strategy could be performed whereby cDNAs encoding F-derivatized peptides of autoantigens and/or allergens are injected instead of the peptides themselves, thus having the same effect as immunizing with peptides themselves. Moreover, the F-derivatized peptides and proteins could be produced by recombinant DNA techniques instead of peptide synthesis of peptide synthesis and the same type of tolerizing immunogen obtained.

Another use of F domain- or F-like domain derivatization of peptides and proteins is to confer upon the derivatized peptide or protein the ability to bind to the cell membrane and enter the cell. The fusion domain or a fusion-like domain could be conjugated to an RNA or DNA molecule as well as a protein to promote entry into cells. The ability of a molecule to enter the cells is important for many molecules to act therapeutically, and can be overcome by addition of the F domain or an F-like domain to the molecule that one wanted to get inside or cells. For example, a powerful inhibitor of cell activation would be a peptide, RNA or DNA species of molecule that competetively bound to an intracellular molecule ncessary for cell activation, but the peptide, RNA or DNA molecule itself did not activate or serve the normal function of the physiologic ligand that it was designed to mimic. Examples of peptide, RNA or DNA molecules that might inhibit cell activation would be molecules that bound to intracellular tyrosine kinases, tyrosine phosphatases, protein Kinase C enzymes or G proteins, just to name a few examples. However, for peptide, RNA or DNA inhibitory ligands to function as cell regulatory agents when administered as therapeutic agents, they must readily bind the cell without killing the cell, and be able to enter the cell and function intracellularly. It has been shown that F-derivatization of the T1-SP10IIIB(A) peptide with the HIV gp41 F domain promotes enhanced binding (Table 13) and entry (Table 14) of the derivatzied T12-SP10IIIB(A) peptide into human B cells. This ability to promote entry of derivatized molecules into the inside of cells represents a novel drug delivery system with potential uses for delivering virtually any type of molecule (RNA, DNA, protein) inside cells for the desired therapeutic effect. For example, F-derivatized proteins of HIV regulatory proteins that might bind to viral RNA but not promote transcription of RNA thus preventing normal binding of HIV transcription factors might be used to treat HIV infections in vivo.

Table 1

| Examples of Autoimmune Diseaes or Disease Model Caused By Autoreactive B Cell Responses | |
|---|---|
| Disease | Pathogenic Antibody Specificity |
| Myasthenia Gravis (MG) | Anti-acetylcholine receptor antibodies cause weakness in MG |
| Juvenile Onset Diabetes Mellitus (Type 1 Diabetes) | Anti-insulin antibodies and anti-islet cell antibodies mediate islet cell destruction |
| Graves' Disease | Anti-thyroid stimulating hormone receptor antibodies mediate the disease |
| Insulin Resistance in Diabetes Mellitus | Anti-insulin antibodies prevent treatant of diabetes with insulin |

Table 2

| Examples Autoimmuine Diseases or Disease Models Caused by Autoreactive T Cell Responses | |
|---|---|
| Experimental autoimmune uveoretinitis (EDU) | T cell responses against retinal S antigen cause eye damage |
| Experimental autoimmune encephalomyelitis (EAE) | T cell responses against myelin basic protein cause neuronal damage |

Table 3

Peptide Sequences Used In Chimpanzee Immunizations

| | |
|---|---|
| F-T1-SP10IIIB(A) | AVGIGALFLGFLKQIINMWQEVGKAMYACTRPNNNTRKSIRIQRGPGRAFVTI |
| T1-SP10IIIB | KQIINMWQEVGKAMYACTRPNNNTRKSIRIQRGPG |
| T1SP10IIIB(A) | KQIINMWQEVGKAMYACTRPNNNTRKSIRIQRGPGRAFVTI |

Table 4

| Tritiated Thymidine Incorporation of Peripheral Blood Mononuclear Cells Following <u>In Vitro</u> Stimulation With HIV Env gp120* | | | |
|---|---|---|---|
| Chimpanzee No. | Immunogen | Pre-Immunization $\Delta$cpm/$10^6$ cells | Post-Immunization (Post/Pre) |
| 884 | T1-SP10IIIB, then T1-SP10IIIB(A) | 169 | 39,189 (232) |
| 1028 | T1-SP10IIIB, then T1-SP10IIIB(A) | 17,955 | 129,121 (7) |
| 1045 | F-T1-SP10IIIB(A) | 6,348 | 12,256 (2) |
| 1070 | F-T1-SP10IIIB(A) | 11,285 | 22,719 (2) |

*Data represent the peak gp120 responses observed during the immunization period. Data for animals 884,1028, and 1045 represent peak responses using from 2ug/ml to 0.5ug/ml of HIVIIIB(LAI) recombinant gp120. Data for animal 1070 represent peak responses using from 1ug/ml to 0.5ug/ml of native HIVIIIB(LAI) gp120.

Table 5

HIV Envelope gp41 Fusion Protein (F) Sequences From Multiple HIV Isolates

| Isolate | Sequence |
|---|---|
| HIV-1 | |
| BH10 | A V G : I G A L F L G F L |
| MN | A A : : - - - - - - - - - |
| SC | - - - T - - - M - - - - - |
| SF2 | - - - I V - - M - - - - - |
| CDC4 | - - - M L - - M - - - - - |
| WMJ2 | - - - T - - - M - - - - - |
| RF | - - - T - - - M - - - - - |
| ELI | - I - : L - - M - - - - - |
| MAL | - I - : L - - M - - - - - |
| Z6 | - I - : L - - M - - - - - |
| Z321 | - I - M : - - F - - - - - |
| JY1 | - I - : L - - V - - - - - |
| WMJ-1 | - - - A - - - M - - - - - |
| HIV-2 | |
| ROD | R G V F V L G F L G F L |
| NIHZ | - - - - - - - - - - - - |

Sequences for BH10 are aa 519-530 from Ratner, L, et al. Nature 313: 277-284, 1985. Sequences for the remainder of the HIV-1 and HIV-2 isolates from Myers, et al. Human Retroviruses and AIDS, 1988, Los Alamos National Laboratory, Los Alamos, New Mexico, p. II-90. WMJ-1 sequence from ref. 18.

## Table 6

### Regions of the TSH Receptor to Which Patient Anti-TSH Receptor Autoantibodies Bind

| Amino Acid No. | Sequence | Ref. |
|---|---|---|
| 333-343 | YVFFKEQEDEI | 17 |
| 12-36 | HQEEDFRVTCKDIQRIPSLPPSTQT | 18 |
| 289-317 | LRQRKSVNALNSPLHQEYEENLGDSIVGY | 18 |
| 352-366 | YYVFFEEQEDEIIGF | 27 |
| 103-111 | YKELPLLKFL | 28 |

Amino acid numbers and sequence from the reference listed

## Table 7

### Examples of Hybrid Peptide Constructs That Could Be Used To Treat Anti-HLA Immune Responses In AIDS

<u>HIV gp120 homology with DP/DQ β chain</u>

gp120 aa261-270 VVSTQLLLNG

HLA DP/DQ aa142-151 VVST*LI*NG

<u>HIV gp41 homology with HLA DR β chain</u>

gp41 aa837-844 EGTDRVI

HLA DR aa19-25 NGTERVR

<u>Hybrid Immunogens</u>: **AVGIGALFLGFLVVSTQLLLNG**
**AVGIGALFLGFLVVSTLING**
**AVGIGALFLGFLEGTDRVI**
**AVGIGALFLGFLNGTERVR**

HIV gp120 and gp41 homologies with HLA Class II are from refs. 25 and 26.

TABLE 8

**Sequences of Synthetic Peptide Constructs Derived From HIV MN and HIVIIIB Env gp120***

| Peptide Name | Peptide Type | Peptide Composition and Sequence (Epitope Type) F T1(Th) SP10(B cell) A(B cell) |
|---|---|---|
| F-T1-SP10IIIB(A) | F-Th-B | AVGIGALFLGFLKQIINMWQEVGKAMYACTRPNNNTRKSIRIQRGPGRAFVTI |
| T1-SP10IIIB(A) | Th-B | KQIINMWQEVGKAMYACTRPNNNTRKSIRIQRGPGRAFVTI |
| T1-SP10IIIB | Th-B | KQIINMWQEVGKAMYACTRPNNNTRKRIRIQRGPG |
| T1-SP10MN(A) | Th-B | KQIINMWQEVGKAMYACTRPNYNKRKRIHIGPGRAFYTTK |

*Each amino acid is represented by a single-letter code that is the first letter of its name, except for arginine (R), asparagine (N), glutamine (Q), glutamic acid (E), lysine (K), phenylalanine (F), tryptophan (W), tyrosine (Y), and aspartic acid (D). F (fusogenic domain) sequence is amino acids 519-530 from HIVIIIB (27). T1 sequence is amino acids 428-443 from HIVIIIB (27). SP10MN(A) sequence is amino acids 301-319 from HIVMN (28). SP10IIIB sequence is amino acids 303-321 from HIVIIIB. (A) sequence is amino acids 320-324 from HIVMN (28) and amino acids 322-327 from HIVIIIB (27).

Th= T helper cell determinant.
B cell = B cell neutralizing antibody determinant.
A = Additional HIV gp120 V3 loop sequences added to the original synthetic peptide (SP10) sequence to add an additional neutralizing and CTL region to the HIV B cell determinant of the hybrid peptide.

27 = Ratner et al, Nature 313:277 (1985)
28 = Myers et al, Human Retroviruses and AIDS (1991), p. III 6-23

Table 19

**Time Course of Anti-Peptide Antibody Responses in Chimpanzees Immunized with HIV Envelope Synthetic Th-B or F-Th-B Peptides**

| Month of Study | Immunogen | Chimpanzee Number 884 | Chimpanzee Number 1028 | Immunogen | Chimpanzee Number 1045 | Chimpanzee Number 1070 |
|---|---|---|---|---|---|---|
| | | Reciprocal of ELISA Titer | | | Reciprocal of ELISA Titer | |
| 1 | | 0 | 0 | | 0 | 0 |
| 2 | Th-B(IIIB) 6mg | 0 | 0 | F-Th-B(IIIB) 6mg | 0 | 0 |
| 3 | Th-B(IIIB) 6mg | 51,200 | 102,400 | F-Th-B(IIIB) 6mg | 0 | 0 |
| 4 | Th-B(IIIB) 6mg | 25,600 | 819,200 | F-Th-B(IIIB) 6mg | 0 | 800 |
| 5 | Th-B(IIIB) 6mg | 25,600 | 204,800* | F-Th-B(IIIB) 6mg | 1,600 | 200 |
| 6 | Th-B(IIIB) 30mg | 51,200 | 102,400 | F-Th-B(IIIB) 30mg | 25,600 | 12,800 |
| 7 | Th-B(IIIB) 30mg | 204,800 | 102,400 | F-Th-B(IIIB) 30mg | 25,600 | 12,800 |
| 8 | Th-B(IIIB) 30mg | 51,200 | 25,600 | F-Th-B(IIIB) 30mg | 6,400 | 12,800 |
| 9 | | 51,200 | 51,200 | | 3,200 | 6,400 |
| 10 | | 12,800 | 25,600 | | 800 | 800 |
| 11 | | 51,200 | 25,600 | | 800 | 1,600 |
| 12 | | 51,200 | 25,600 | | 1,600 | 800 |
| 13 | | 25,600 | 25,600 | | 200 | 200 |
| 14 | Th-B(IIIB) 6mg | 51,200 | 25,600 | F-Th-B(IIIB) 1mg | 200 | 400 |
| 15 | | 102,400 | 12,800 | | 800 | 800 |
| 16 | Th-B(MN) 6mg | 25,600 | 12,800 | Th-B(IIIB) 6mg | 100 | 0 |
| 17 | Th-B(MN) 6mg | 12,800 | 3,200 | Th-B(MN) 6mg | 1,600 | 3,200 |
| 18 | | 25,600 | 6,400 | | 6,400 | 25,600 |
| 19 | Th-B(MN) 6mg | 25,600 | 1,600 | Th-B(MN) 6mg | 6,400 | 51,200 |
| 20 | | 51,200 | 6,400 | Th-B(MN) 6mg | 51,200 | 102,400# |

Titers are endpoint ELISA titers (titers at which E/C were ≥ 3.0) against the Th-B peptide, T1-SP10IIIB.
* Animal 1028 did not receive the month 5 injection due to a sterile abscess at the injection site. All injections in animal 1028 after month 5 were in PBS alone.
# Animal 1070 did not receive the month 20 immunization due to the presence of high levels of anti-HIV neutralize antibodies.
For animals 884 and 1028, immunizations at months 2-5 were with T1-SP10IIIB, months 6,7,8 and 14, T1-SP10IIIB(A). For animals 1045 and 1070 immunization at month 16 was with T1-SP10IIIB(A).

Table 10

**Mean Lymphocyte and Lymphocyte Subset Levels In Chimpanzees Before and During Immunization With HIV Envelope Synthetic Peptides***

| Leukocyte Subset | Chimpanzee Number | | | | | | | | | | | |
|---|---|---|---|---|---|---|---|---|---|---|---|---|
| | 884 | | | 1028 | | | 1045 | | | 1070 | | |
| | Before | During | % Change | Before | During | % Change | Before | During | % Change | Before | During | % Change |
| | Cells/mm³ ± SEM | | | | | | | | | | | |
| Total Lymphocytes | 4034±452 | 3046±249 | -26% | 3164±396 | 3286±660 | +4% | 3164±397 | 1426±116 | -55%† | 3943±885 | 2768±296 | -30% |
| T cells | 2629±384 | 2054±178 | -24% | 2565±276 | 2027±402 | -21% | 2460±253 | 1012±82 | -59%† | 3337±762 | 1887±184 | -44%‡ |
| B cells | 356±47 | 365±39 | +3% | 411±103 | 458±47 | +11% | 293±32 | 175±15 | -40%§ | 302±53 | 232±22 | -23% |
| NK cells | 345±82 | 317±43 | -9% | 257±25 | 434±128 | +68% | 112±27 | 61±7 | -45%‡ | 478±148 | 306±44 | -36% |

*"Before" samples were studied over a 5 month period prior to immunization with peptides; n = 5 for lymphocytes, n = 3 for T cells, B cells and NK cells. "During" samples were taken from months 2-14 of immunization; n = 11 for lymphocytes, T,B, and NK cells. Unless noted, p values for percent change comparing "before" values with "during" values was not significant with p> .05 using student's t test.

† = p> .001

‡ = p> .02

§ = p> .005

Table 11

Neutralization of HIV LAI/IIIB and HIV MN in Syncytium Inhibition Assay in Chimpanzees Immunized with T1-SP10 Peptides

| Animal No. | Month 18 | | Month 19 | | Month 20 | |
|---|---|---|---|---|---|---|
| | LAI/IIIB | MN | LAI/IIIB | MN | LAI/IIIB | MN |
| | Presence of Neutralization in Syncytium Inhibition Assay (Reciprocal Titer in RT Inhibition Assay) | | | | | |
| 884 | - | - (20) | - | - | - | - (24) |
| 1028 | - | - | - | - | - | - |
| 1045 | - | - (23) | +/- (23) | - (23) | - (22) | - (24) |
| 1070 | +/- (92) | - (22) | + (100) | + (96) | +/- (86) | ++ (350) |

- = < 48% inhibition of syncytia.
+/- = ≥ 49% and < 80% inhibition of syncytia.
+ = ≥ 80% inhibition of syncytia, titer 1:10.
++ = ≥ 80% inhibition of syncytia, titer 1:20.

Table 12

Reactivity of Chimpanzee Serum with Truncated Forms of the Th-B Peptide T1-SP10IIIB#

| Chimpanzee No. (Bleed Date) | T1-SP10IIIB | T1-flu | SP10C | SP10D | SP10E |
|---|---|---|---|---|---|
| | Endpoint Titer (> 3.0 E/C) In ELISA Assay | | | | |
| 884 (Month 7) | 204,800 | 800 | > 102,400* | 51,200 | 3,200 |
| 1028 (Month 7) | 102,400 | 800 | 102,400 | 51,200 | 3,200 |

(Column heading: Peptide Used in ELISA Binding Assay)

#Peptides used in ELISA Assay were:

T1-SP10IIIB - KQIINMWQEVGKAMYACTRPNNNTRKSIRIQRGPG
T1-flu - KQIINMWQEVGKAMYATYQRTRALVTG
SP10C - (C)TRKSIRIQRGPGR(Y)
SP10D - (C)IRIQRGPGR
SP10E - (C)TRPNNNTRKSIR

ELISA assay performed as described in Methods.
Flu sequence (TYQRTRALVTG) is from influenza nucleoprotein, strain A PR/8/34 from Deres et al, Nature 342:561 (1989).

*E/C at 1:102,400 = 6.0.

Table 13

| Effect of Derivatizing T1-SP10IIIB(A) Peptide With the HIV gp41 Fusogenic (F) Domain on Peptide Ability to Bind to Human Cells | | | |
|---|---|---|---|
| Peptide | Antibody | MFC 4 Degrees C, 1 Hr. | MFC 37 Degrees C, 21 Hr. |
| None | Anti-gp120 | 7.6 | 13.6 |
| T1-SP10IIIB(A) 10ug/ml | Anti-gp120 | 14.7 | 14.0 |
| F1-T1-SP10IIIB(A) | Anti-gp120 | 82.8 | 36.7 |
| Anti-gp120 momoclonal antibody was 0.5beta from the NIAID AIDS Research and Reference Reagent Program (Matsushita et al J. Virol. 62:2107, 1988). Cells used were human JY B cells which were incubated either for 1 hour at 4 degrees C or for 21 hours at 37 degrees C and then reacted with saturating amounts of the anti-gp120IIIB mab, 0.5beta followed by FITC-conjugated goat anti-mouse Ig reagent. The amount of fluoresence was determined on a flow cytometer and fluoresence brightness was expressed as MFC=mean channel fluoresence. | | | |

Table shows that conjugation of the F domain on the T1-SP10IIIB(A) peptide confers on it the ability to bind to JY B cells better that the T1-SP10IIIB(A) peptide alone, and that after incubation at 37 degrees C, the F-T1-SP10IIIB(A) peptide is decreased on the surface of the cells.

Table 14

| Reactivity of anti-gp120 Monoclonal Antibody with Acetone-Fixed JY B Cells That Had Been Incubated With F-T1-SP10IIIB(A) Peptide (10µg/ml) For 21 Hours at 37 Degrees C | | |
|---|---|---|
| Peptide | Antibody | % Intracytoplasmic Positive |
| T1-SP10IIIB(A) | Control | 0 |
| T1-SP10IIIB(A) | Anti-gp120 | 0 |
| F-T1-SP10IIIB(A) | Control | 0 |
| F-T1-Sp10IIIB(A) | Anti-gp120 | 76 faint, 24 bright |

Cells were incubated as descirbed in Table 13. After 21 hours at 37 degrees C, cytocentrifuge preparations of cells were prepared, acetone fixed, and reacted either with control mab P3x63 Ag8 or with anti-gp120 mab 0.5beta. Slides were read for either faint or bright intracytoplasmic fluoresence on a fluoresence microscope. Data show that after incubation of 10 ug/ml of peptide for 21 hours at 37 degrees C, the F-T1-SP10IIIB(A) peptide could be detected inside the JY B cells whereas the T1-SP10MN(A) peptide could not be detected.

**Claims**

1. Use of a 2 to 20 amino acid hydrophobic peptide linked to the N-terminus or C-terminus of an immunogenic peptide or protein for preparing a synthetic immune system toleragen capable of inducing immune tolerance in a mammal.

2. The use according to claim 1, wherein the hydrophobic peptide comprises 5 to 15 amino acids.

3. The use according to claim 2, wherein the hydrophobic peptide comprises 7 to 13 amino acids.

4. The use according to anyone of claims 1 to 3 wherein the hydrophobic peptide is a segment from a HIV or HIV-related virus protein.

5. The use according to claim 4 wherein the hydrophobic peptide is AVGIGALFLGFL.

6. The use according to anyone of claims 1 to 5 wherein the immunogenic peptide or protein is an acetylcholine receptor protein.

7. The use according to anyone of claims 1 to 5, wherein the immunogenic peptide or protein is a fragment of an acetylcholine receptor protein.

8. The use according to anyone of claims 1 to 5 wherein the immunogenic peptide or protein is an insulin protein, or fragment thereof.

9. The use according to anyone of claims 1 to 5 wherein the immunogenic peptide or protein is a TSH receptor protein, or fragment thereof.

10. The use according to anyone of claims 1 to 5 wherein the immunogenic peptide or protein is an autoimmune T cell antigen, or fragment thereof.

11. The use according to anyone of claims 1 to 5 wherein the immunogenic peptide or protein is a retinal S protein, or fragment thereof

12. The use according to anyone of claims 1 to 5 wherein the immunogenic peptide or protein is a B cell determinant or a protein that induces pathogenic B cell antibody production in an autoimmune or inflammatory disease.

13. The use according to anyone of claims 1 to 12 wherein the hydrophobic part of the toleragen is any hydrophobic peptide from a transmembrane region of a transmembrane protein, or is a random mix of hydrophobic amino acids.

**14.** The use according to anyone of claims 1 to 13 wherein said mammal is a primate.

**15.** The use according to claim 14, wherein said primate is a human.

**16.** An hydrophobic peptide having the sequence AVGIGALFLGFL.

**Patentansprüche**

**1.** Verwendung eines hydrophoben, 2 bis 20 Aminosäuren aufweisenden Peptids, welches an den N-Terminus oder den C-Terminus eines immunogenen Peptids oder Proteins gebunden ist, zur Herstellung eines synthetischen Immunsystemtoleragens, welches zur Hervorrufung von Immuntoleranz bei einem Säugetier fähig ist.

**2.** Verwendung nach Anspruch 1, worin das hydrophobe Peptid 5 bis 15 Aminosäuren umfaßt.

**3.** Verwendung nach Anspruch 2, worin das hydrophobe Peptid 7 bis 13 Aminosäuren umfaßt.

**4.** Verwendung nach einem der Ansprüche 1 bis 3, worin das hydrophobe Peptid ein Segment aus einem HIV- oder einem HIV-verwandten Virusprotein ist.

**5.** Verwendung nach Anspruch 4, worin das hydrophobe Peptid AVGIGALFLGFL ist.

**6.** Verwendung nach einem der Ansprüche 1 bis 5, worin das immunogene Peptid oder Protein ein Acetylcholin-Rezeptor-Protein ist.

**7.** Verwendung nach einem der Ansprüche 1 bis 5, worin das immunogene Peptid oder Protein ein Fragment von einem Acetylcholin-Rezeptor-Protein ist.

**8.** Verwendung nach einem der Ansprüche 1 bis 5, worin das immunogene Peptid oder Protein ein Insulinprotein oder ein Fragment hievon ist.

**9.** Verwendung nach einem der Ansprüche 1 bis 5, worin das immunogene Peptid oder Protein ein TSH-Rezeptor-Protein oder ein Fragment hievon ist.

**10.** Verwendung nach einem der Ansprüche 1 bis 5, worin das immunogene Peptid oder Protein ein Autoimmun-T-Zell-Antigen oder ein Fragment hievon ist.

**11.** Verwendung nach einem der Ansprüche 1 bis 5, worin das immunogene Peptid oder Protein ein Retinal-S-Protein oder ein Fragment hievon ist.

**12.** Verwendung nach einem der Ansprüche 1 bis 5, worin das immunogene Peptid oder Protein eine B-Zell-Determinante oder ein Protein ist, welches eine pathogene B-Zell-Antikörperbildung bei einer Autoimmunerkrankung oder einer entzündlichen Erkrankung hervorruft.

**13.** Verwendung nach einem der Ansprüche 1 bis 12, worin der hydrophobe Teil des Toleragens jedes beliebige hydrophobe Peptid aus einer Transmembranregion eines Transmembranproteins ist, oder ein zufälliges Gemisch hydrophober Aminosäuren ist.

**14.** Verwendung nach einem der Ansprüche 1 bis 13, worin das genannte Säugetier ein Primat ist.

**15.** Verwendung nach Anspruch 14, worin der genannte Primat ein Mensch ist.

**16.** Hydrophobes Peptid mit der Sequenz AVGIGALFLGFL.

**Revendications**

**1.** Utilisation d'un peptide hydrophobe, constitué par 2 à 20 acides aminés, lié à la terminaison N ou à la terminaison C d'un peptide ou protéine immunogène, pour la préparation d'un système immun synthétique toléragène, capable d'induire une tolérance immunitaire chez un mammifère.

2. Utilisation selon la revendication 1, dans laquelle le peptide hydrophobe comprend de 5 à 15 acides aminés.

3. Utilisation selon la revendication 2, dans laquelle le peptide hydrophobe comprend de 7 à 13 acides aminés.

4. Utilisation selon l'une quelconque des revendications 1 à 3, dans laquelle le peptide hydrophobe est un segment d'une protéine du virus HIV ou d'un virus apparenté au HIV.

5. Utilisation selon la revendication 4, dans laquelle le peptide hydrophobe est AVGIGALFLGFL.

6. Utilisation selon l'une quelconque des revendications 1 à 5, dans laquelle le peptide ou protéine immunogène est une protéine du récepteur de l'acétylcholine.

7. Utilisation selon l'une quelconque des revendications 1 à 5, dans laquelle le peptide ou protéine immunogène est un fragment d'une protéine du récepteur de l'acétylcholine.

8. Utilisation selon l'une quelconque des revendications 1 à 5, dans laquelle le peptide ou protéine immunogène est une protéine d'insuline, ou un fragment de celle-ci.

9. Utilisation selon l'une quelconque des revendications 1 à 5, dans laquelle le peptide ou protéine immunogène est une protéine récepteur de TSH, ou un fragment de celle-ci.

10. Utilisation selon l'une quelconque des revendications 1 à 5, dans laquelle le peptide ou protéine immunogène est un antigène de lymphocyte T autoimmun, ou un fragment de celui-ci.

11. Utilisation selon l'une quelconque des revendications 1 à 5, dans laquelle le peptide ou protéine immunogène est une protéine rétinal S, ou un fragment de celle-ci.

12. Utilisation selon l'une quelconque des revendications 1 à 5, dans laquelle le peptide ou protéine immunogène est un déterminant de lymphocytes B ou une protéine qui provoque la production pathogène d'anticorps de lymphocytes B dans une maladie autoimmune ou inflammatoire.

13. Utilisation selon l'une quelconque des revendications 1 à 12, dans laquelle la partie hydrophobe du toléragène est un peptide hydrophobe quelconque issu d'une région transmembranaire d'une protéine transmembranaire, ou est un mélange aléatoire d'acides aminés hydrophobes.

14. Utilisation selon l'une quelconque des revendications 1 à 13, dans laquelle ledit mammifère est un primate.

15. Utilisation selon la revendication 14, dans laquelle ledit primate est un être humain.

16. Peptide hydrophobe comprenant la séquence AVGIGALFLGFL.

FIG. 1A

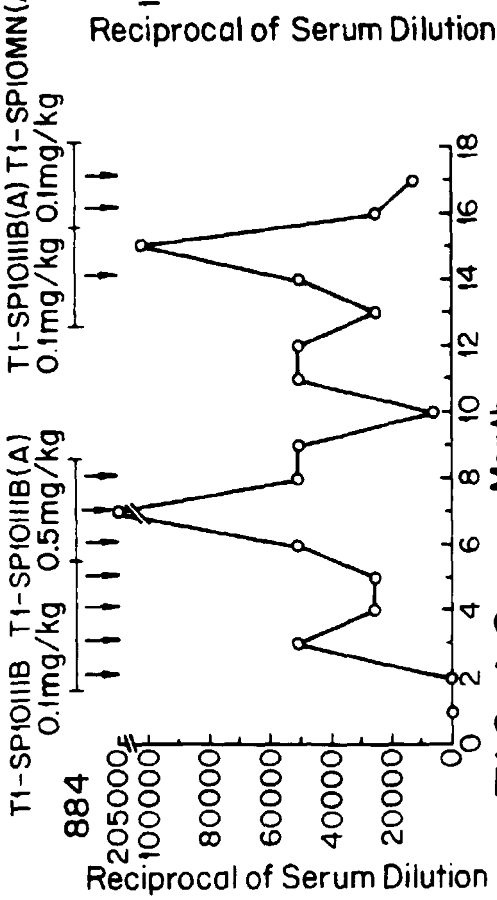


FIG. 1B

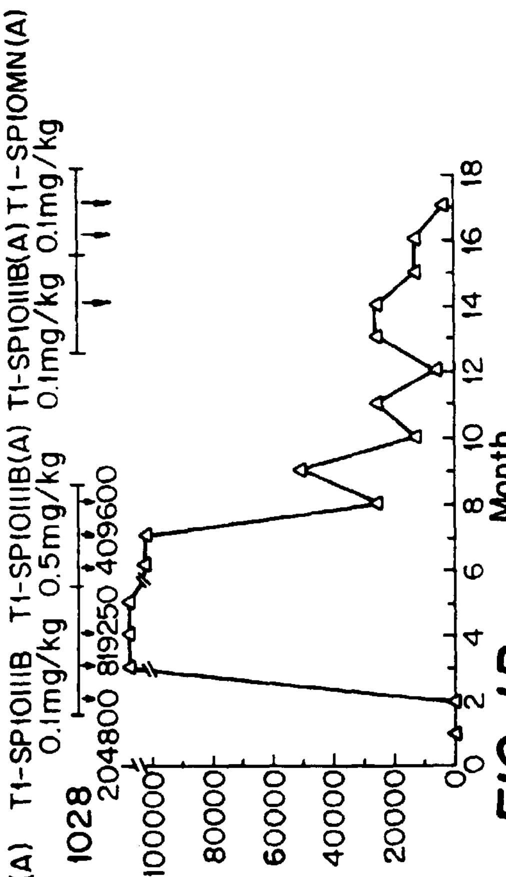


FIG. 1C

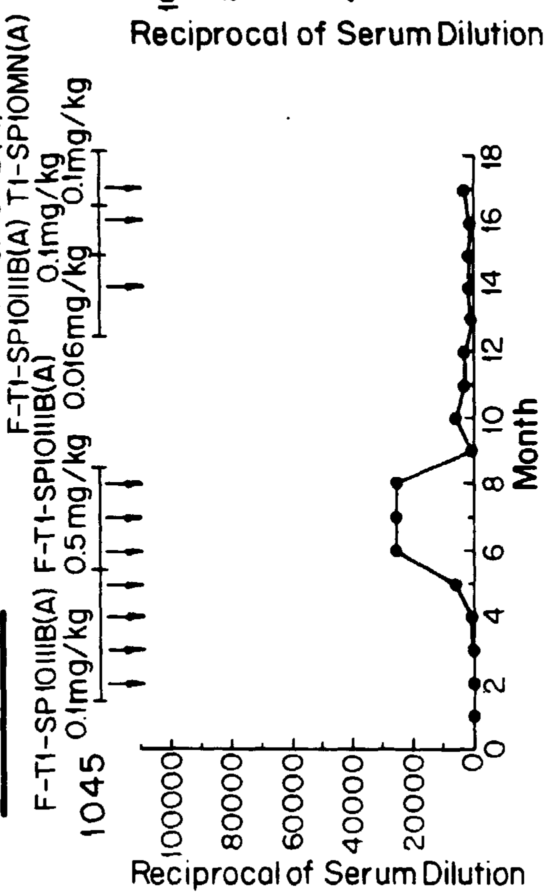


FIG. 1D

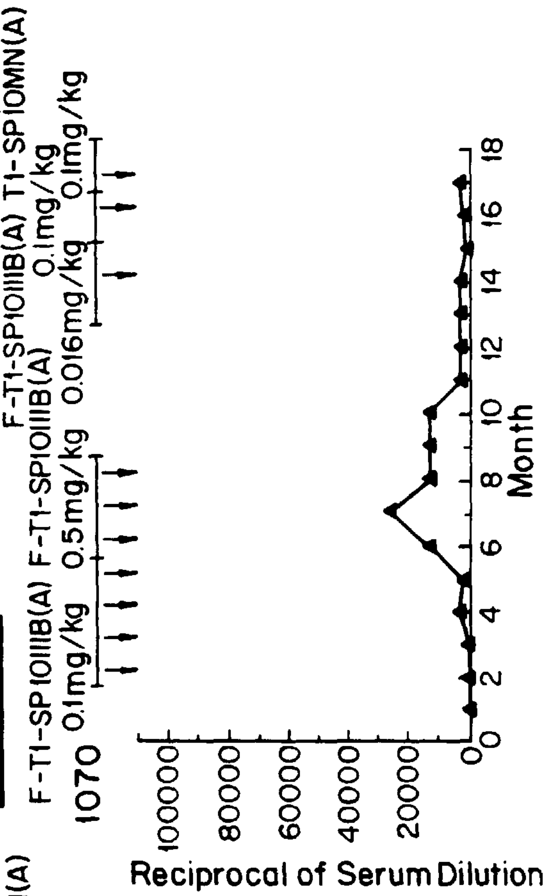

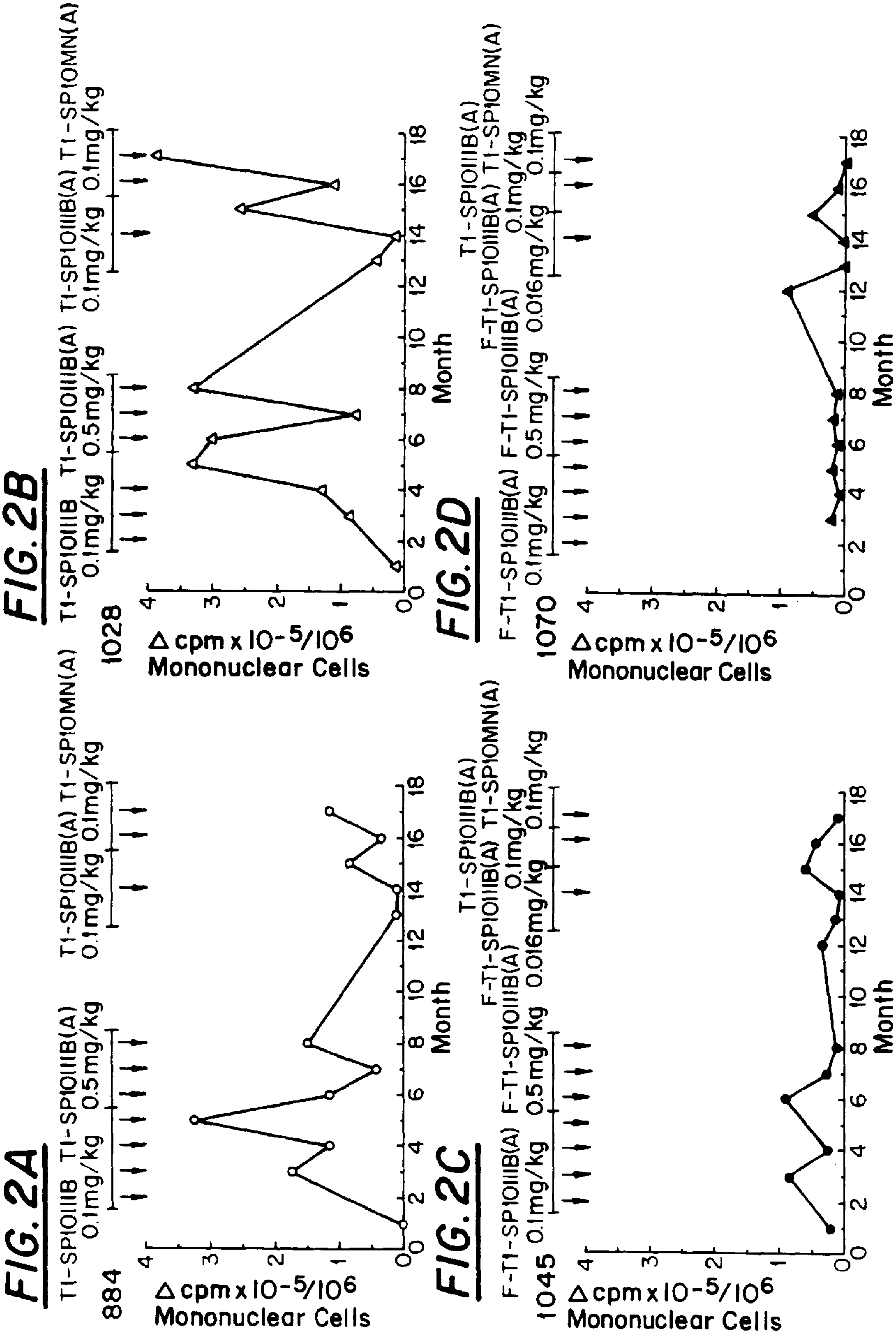


FIG. 2A

FIG. 2B

FIG. 2C

FIG. 2D

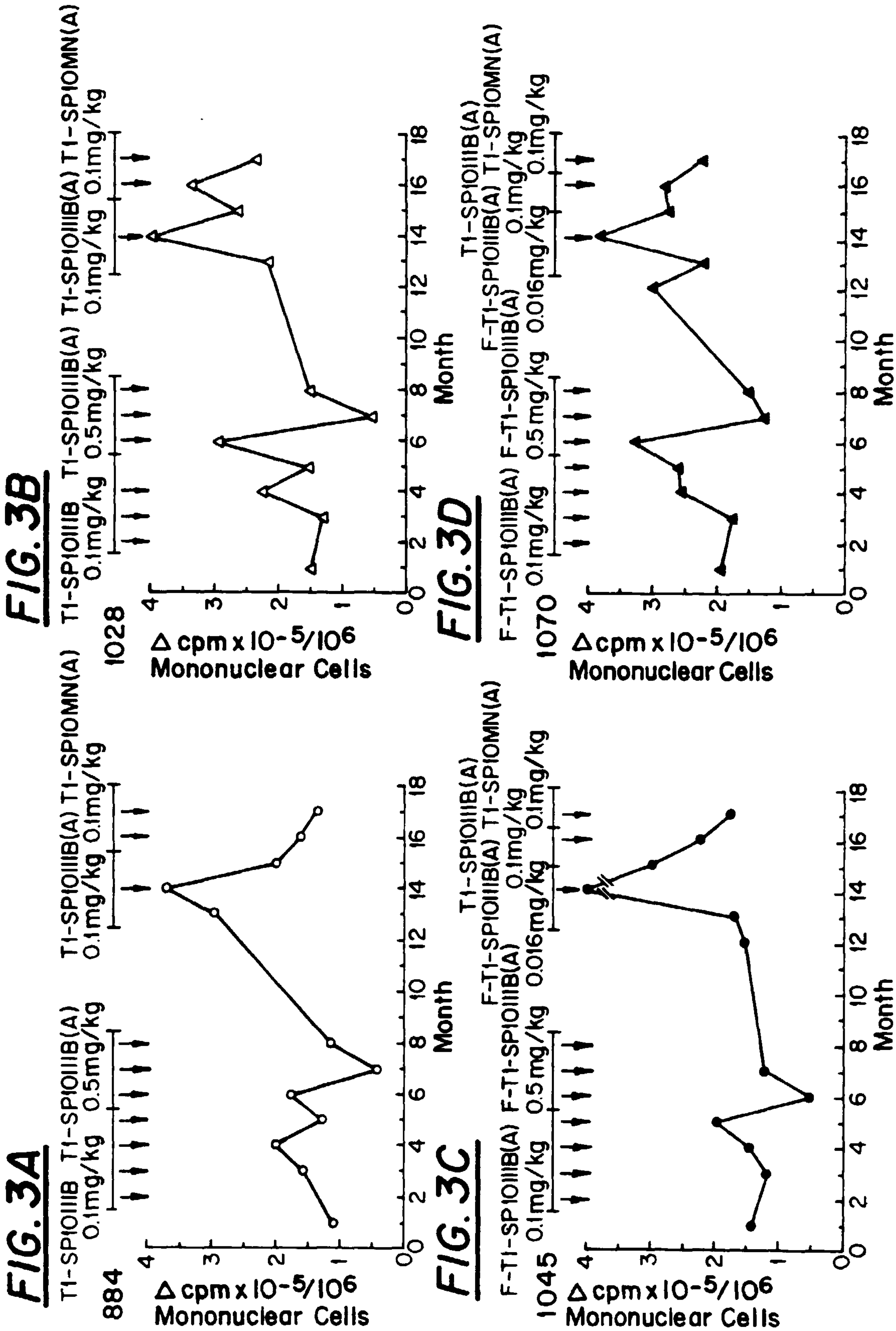


FIG. 3A

FIG. 3B

FIG. 3C

FIG. 3D

FIG. 4

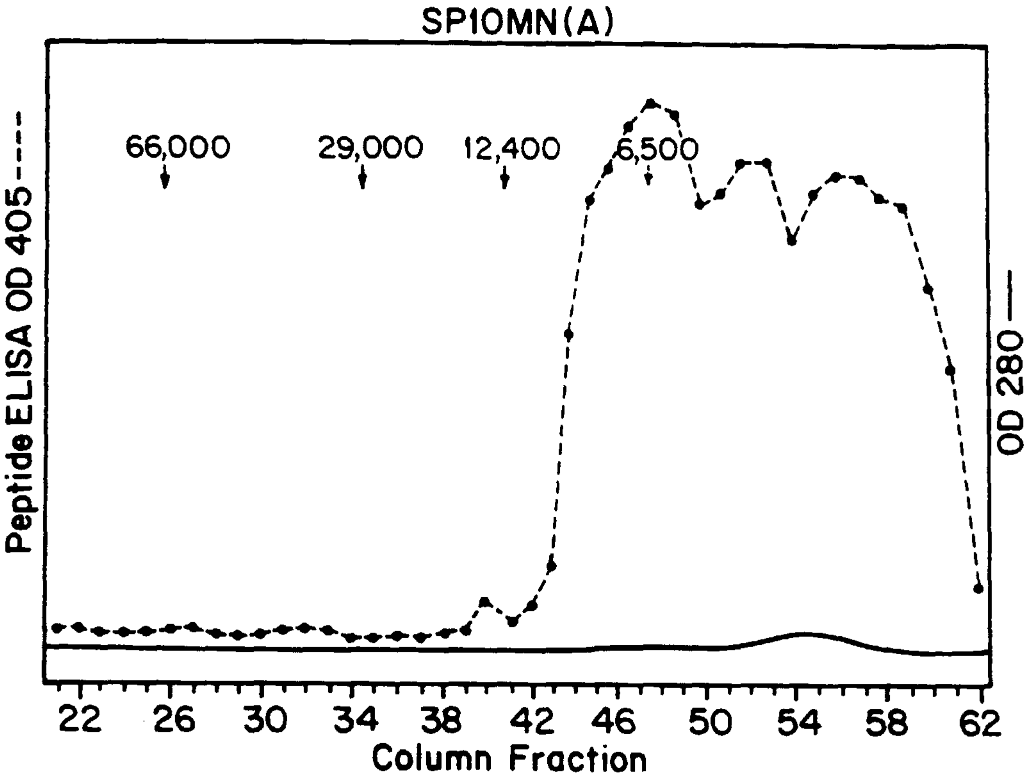
SP1OMN(A)
66,000
29,000
12,400
6,500
Peptide ELISA OD 405
OD 280
22 26 30 34 38 42 46 50 54 58 62
Column Fraction

FIG. 5

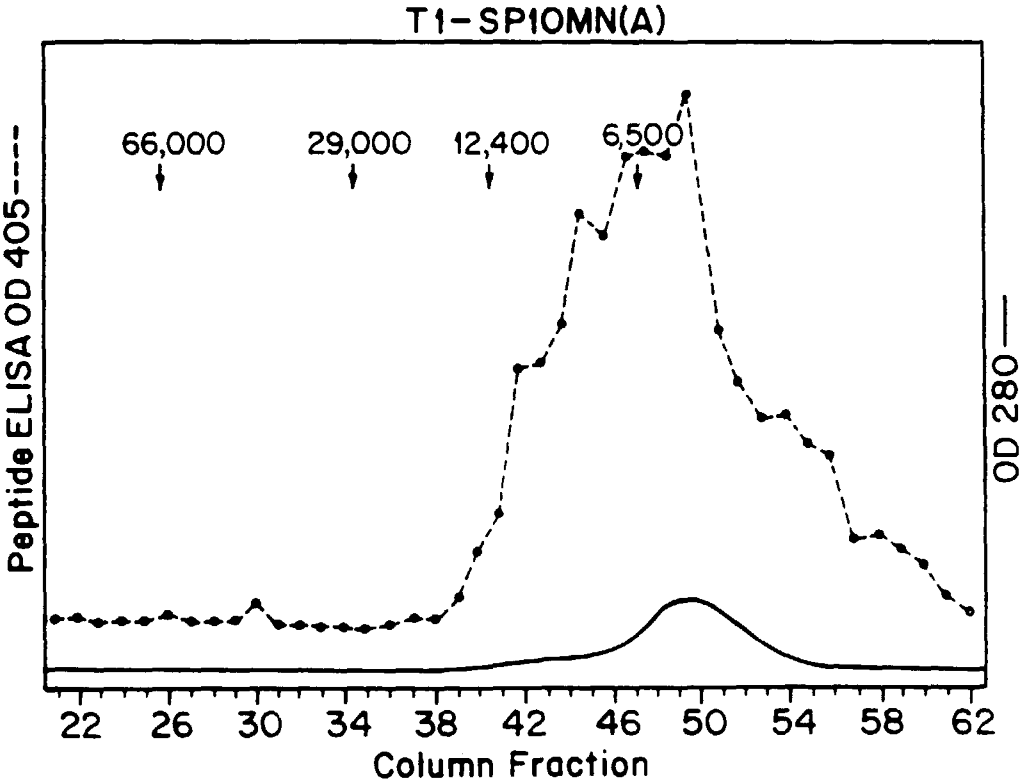
T1-SP1OMN(A)
66,000
29,000
12,400
6,500
Peptide ELISA OD 405
OD 280
22 26 30 34 38 42 46 50 54 58 62
Column Fraction

FIG. 6

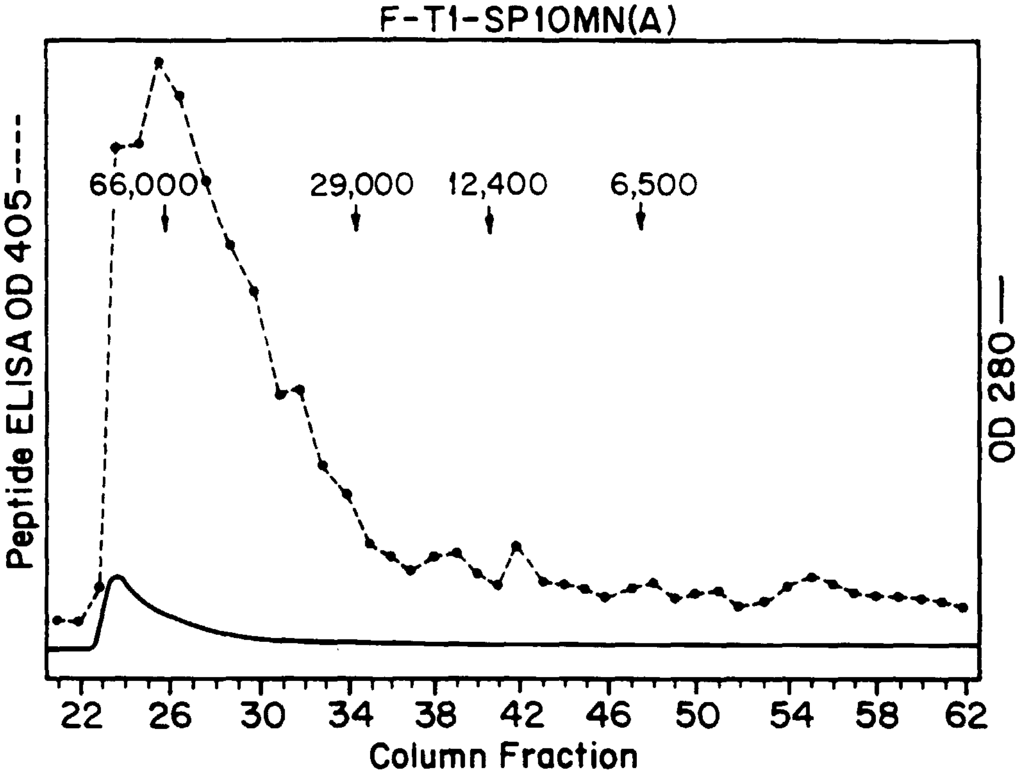
F-T1-SP10MN(A)
66,000
29,000
12,400
6,500
Peptide ELISA OD 405
OD 280
22
26
30
34
38
42
46
50
54
58
62
Column Fraction

FIG. 7

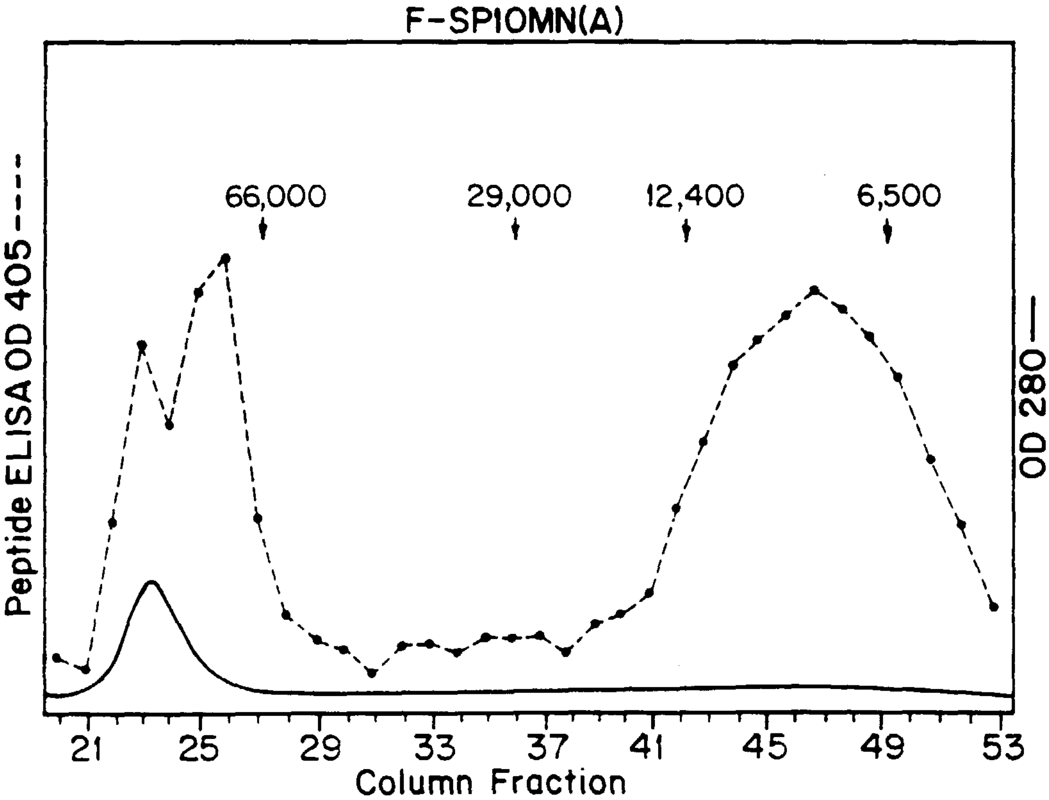
F-SP10MN(A)
66,000
29,000
12,400
6,500
Peptide ELISA OD 405
OD 280
21
25
29
33
37
41
45
49
53
Column Fraction

FIG. 8

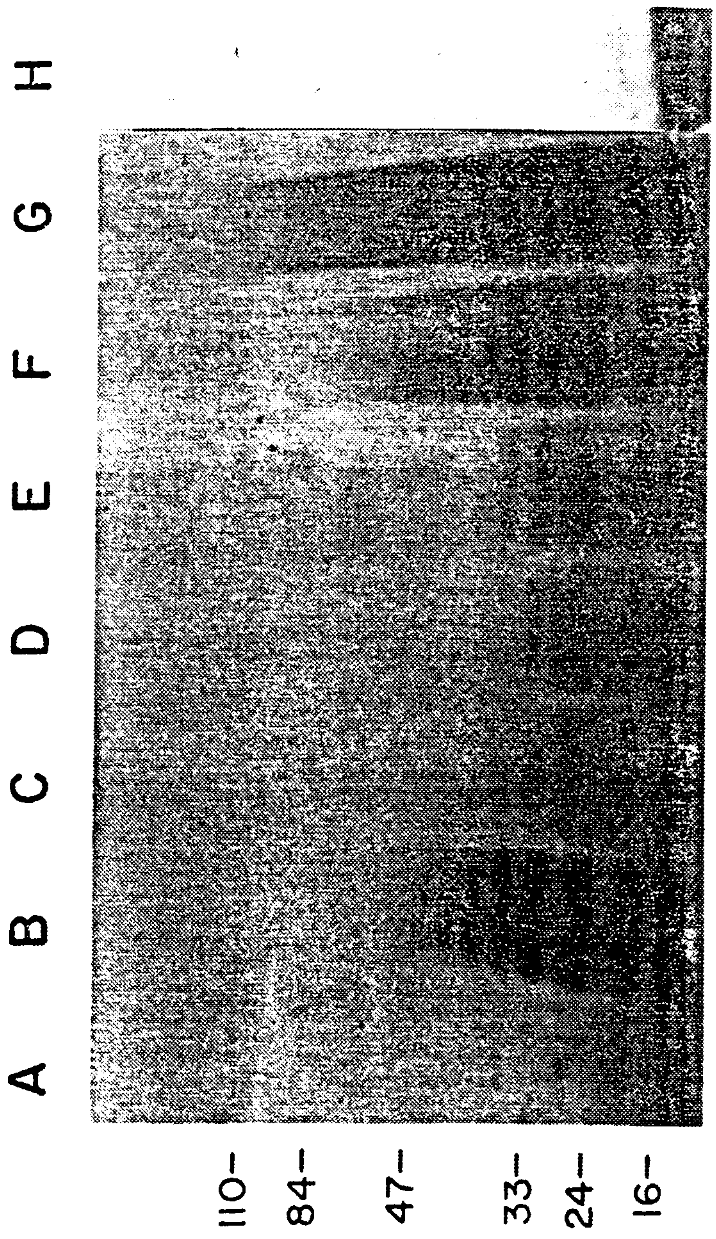

FIG. 9

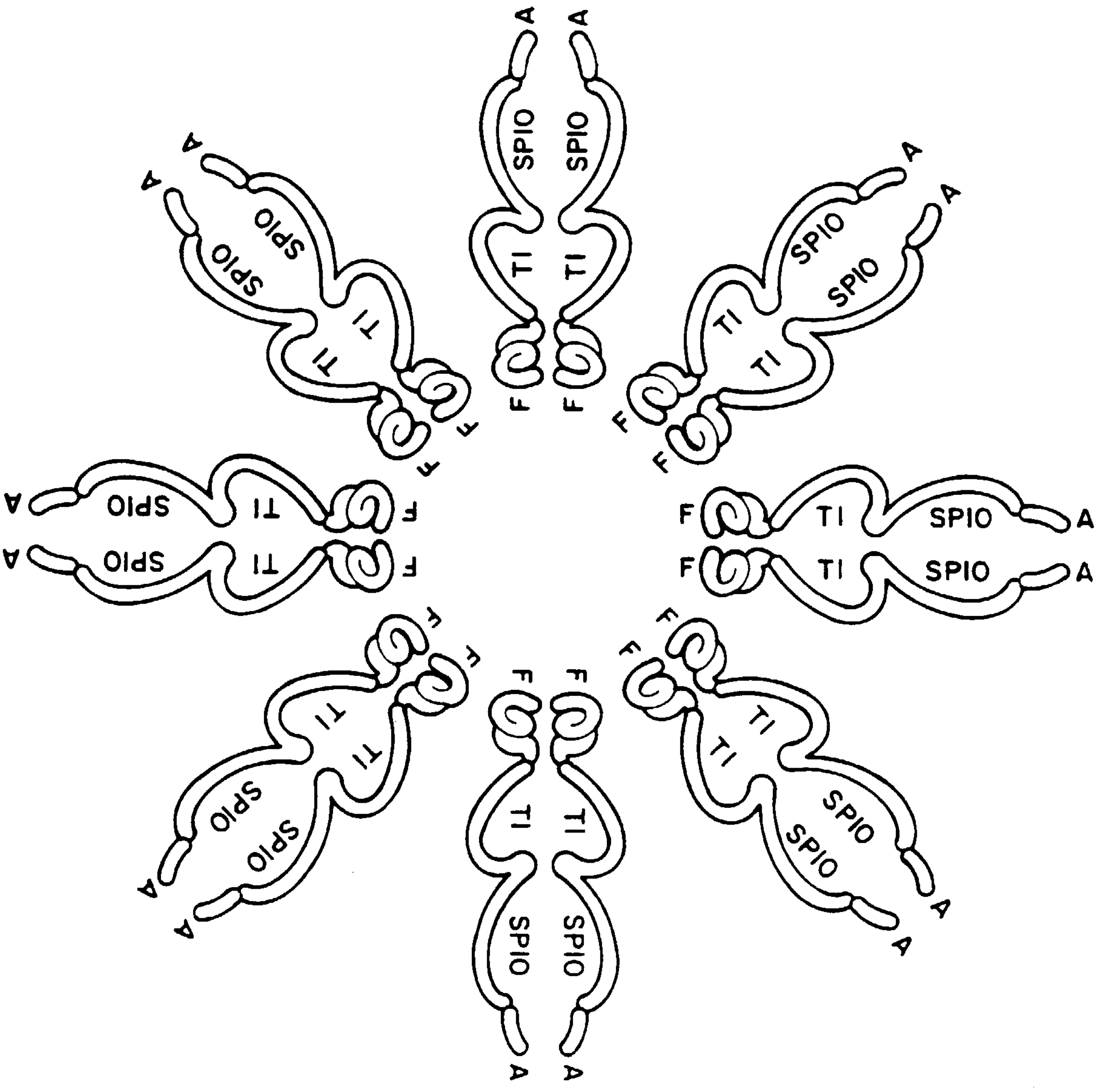

# FIG. 10

Envelope Sequences

| Peptide Name | Region (F / T1 / SP10 / A) |
| --- | --- |
| HIV MN | |
| T1-SP10MN | KQIINMWQEVGKAMYACTRPNYNKRKRIHIGPGRA |
| T1-SP10MN(A) | KQIINMWQEVGKAMYACTRPNYNKRKRIHIGPGRA FYTTK |
| F-T1-SP10MN | AVGIGALFLGFLKQIINMWQEVGKAMYACTRPNYNKRKRIHIGPGRA |
| F-T1-SP10MN(A) | AVGIGALFLGFLKQIINMWQEVGKAMYACTRPNYNKRKRIHIGPGRA FYTTK |

## FIG. 10 cont.

| | |
|---|---|
| F-SP10MN(A) | AVGIGALFLGFL.................CTRPNYNKRKRIHIGPGRA FYTTK |
| SP10MN(A) | CTRPNYNKRKRIHIGPGRA FYTTK |
| HIVIIIB | |
| T1-SP10IIIB(A) | KQIINMWQEVGKAMYACTRPNNNTRKSIRIQRGPG RAFVTI |
| F-T1- SP10IIIB(A) | AVGIGALFLGFLKQIINMWQEVGKAMYACTRPNNNTRKSIRIQRGPG RAFVTI |

FIG. 11A

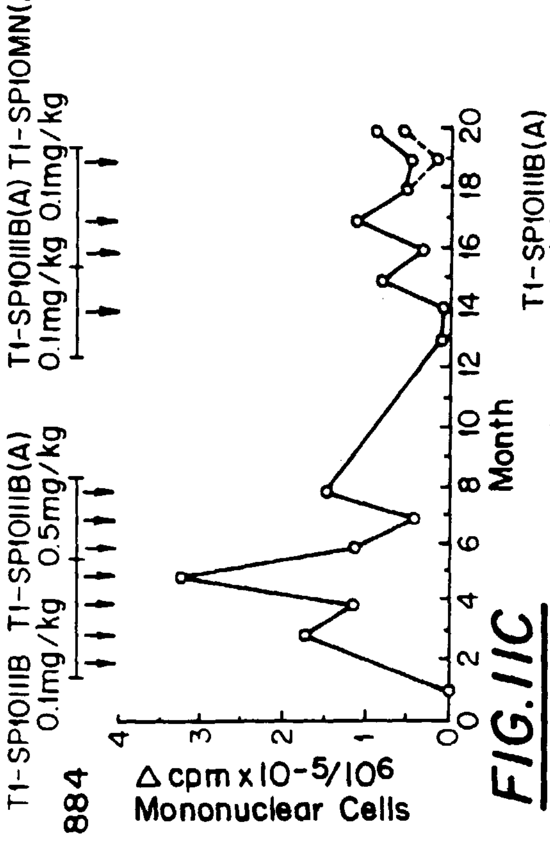


FIG. 11B

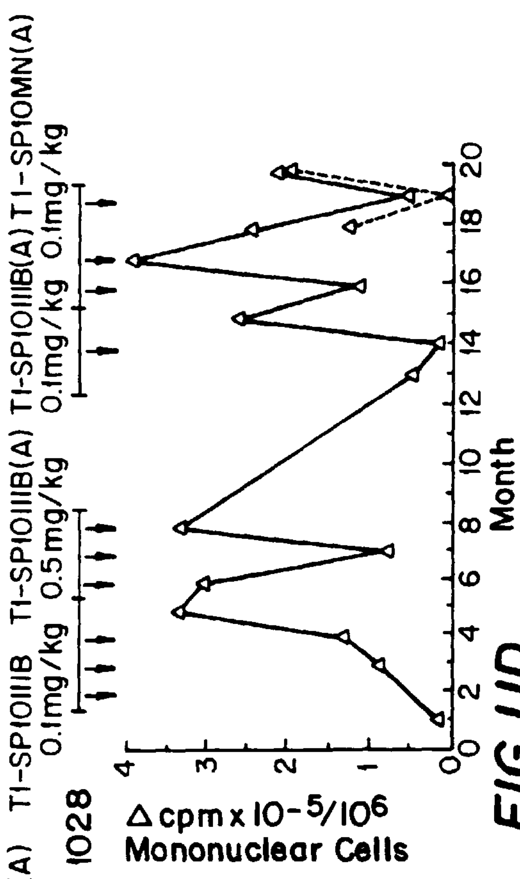


FIG. 11C

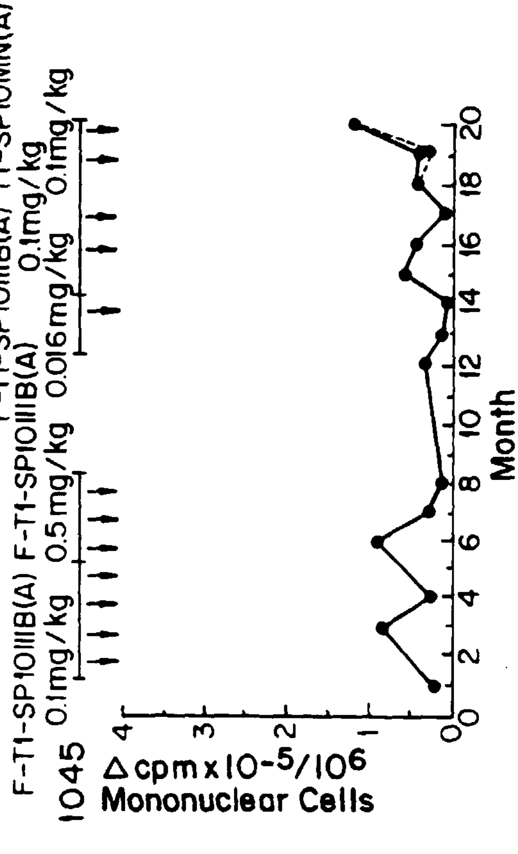


FIG. 11D

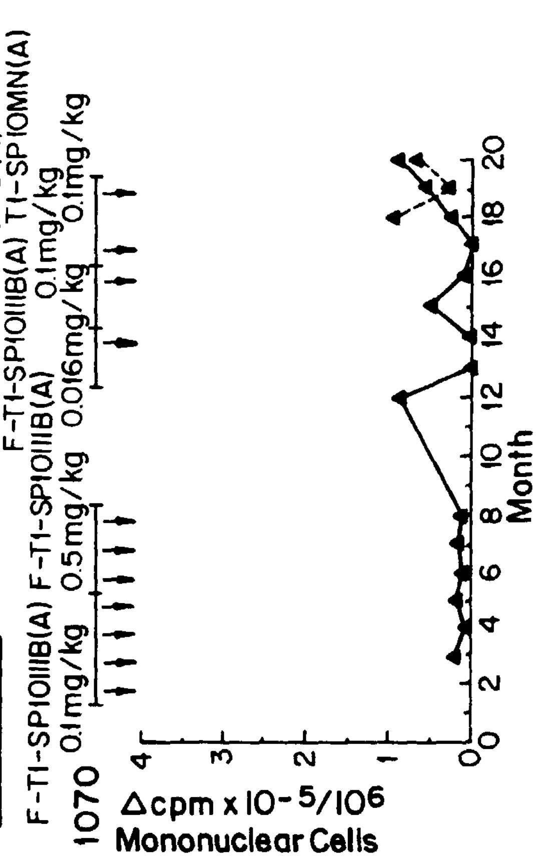

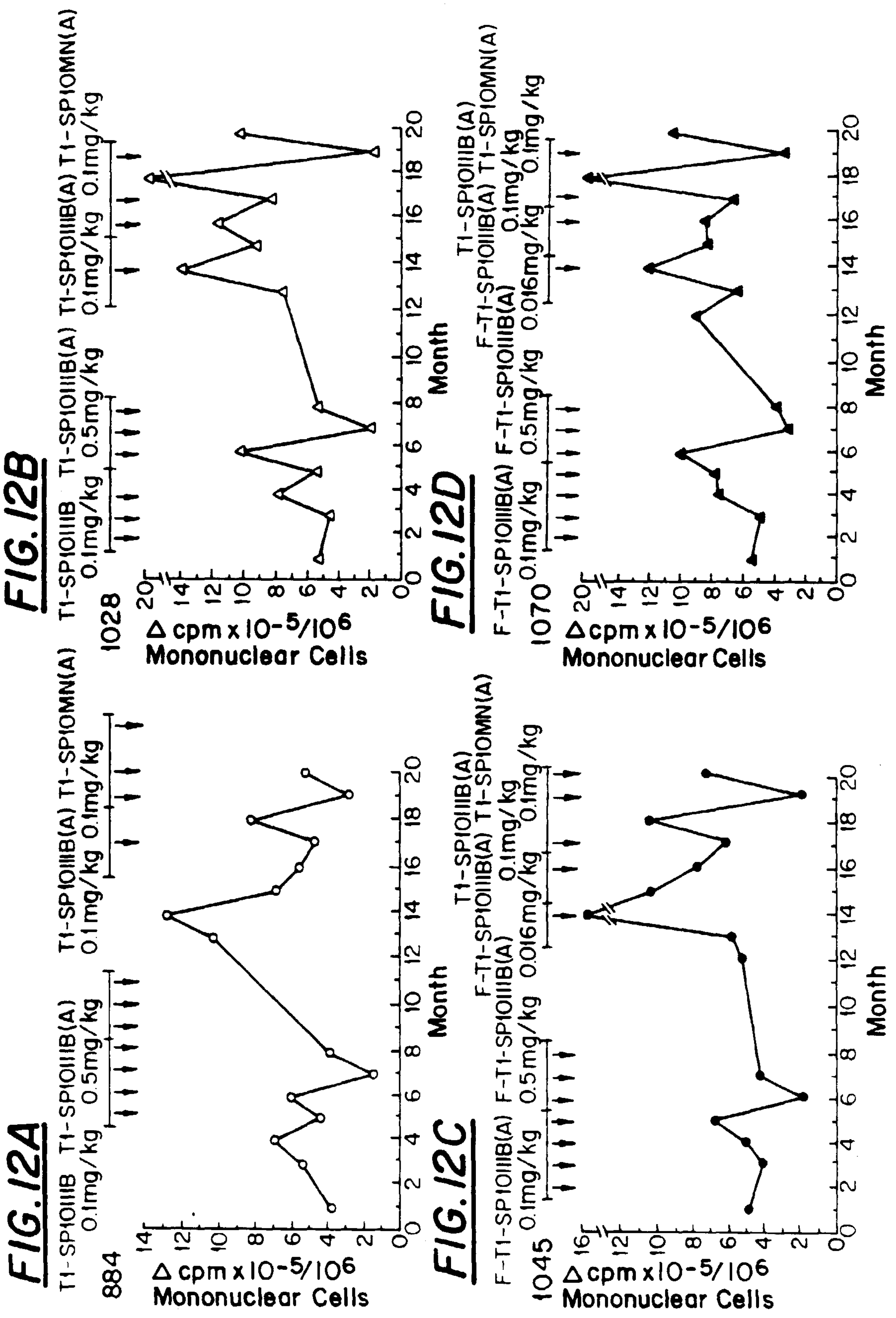


FIG. 12A

FIG. 12B

FIG. 12C

FIG. 12D

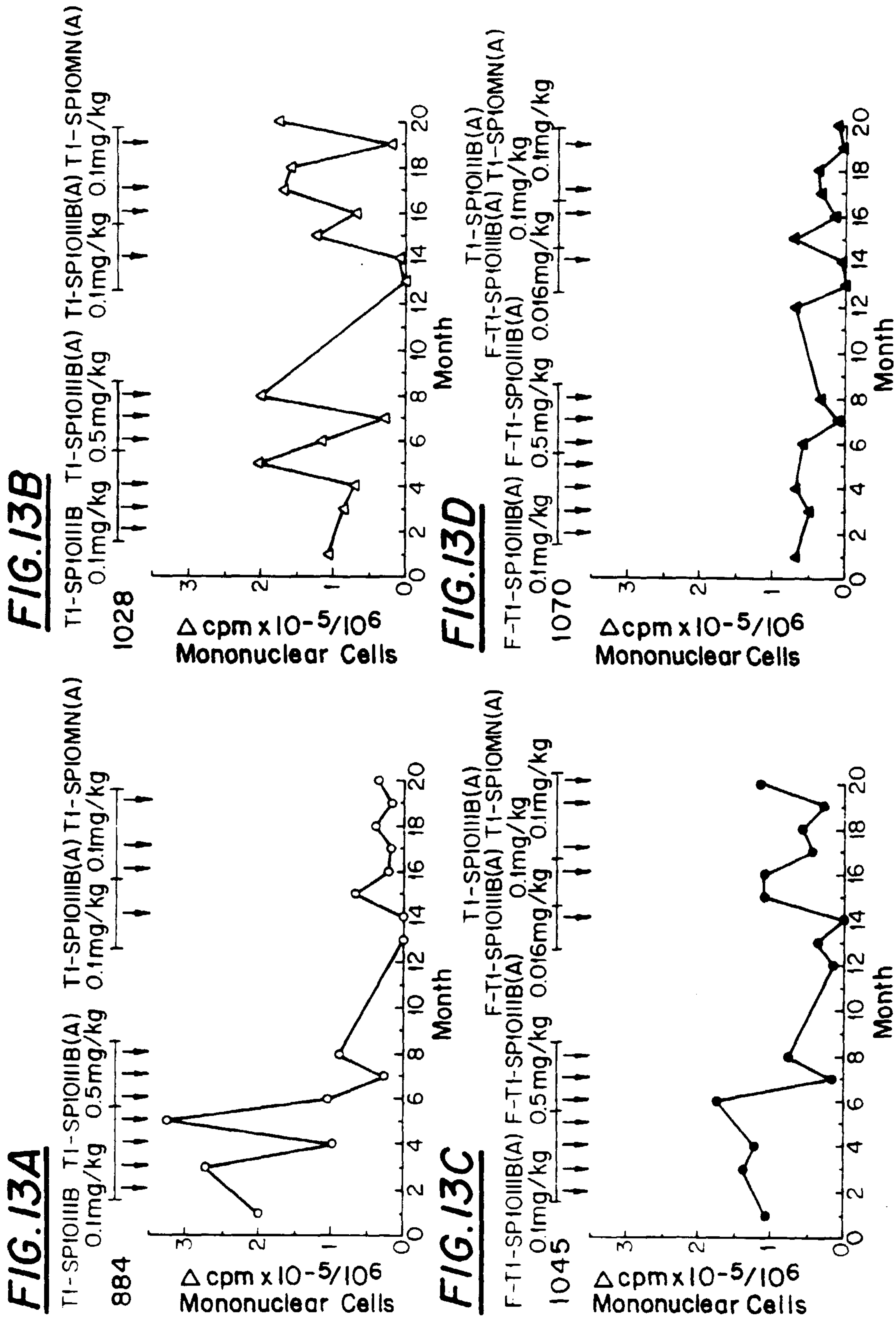
FIG. 13A
884
T1-SP10IIIB 0.1mg/kg
T1-SP10IIIB(A) 0.5mg/kg
T1-SP10IIIB(A) 0.1mg/kg
T1-SP10MN(A) 0.1mg/kg
Δ cpm x 10-5/106
Mononuclear Cells
Month
FIG. 13B
1028
T1-SP10IIIB 0.1mg/kg
T1-SP10IIIB(A) 0.5mg/kg
T1-SP10IIIB(A) 0.1mg/kg
T1-SP10MN(A) 0.1mg/kg
Δ cpm x 10-5/106
Mononuclear Cells
Month
FIG. 13C
1045
F-T1-SP10IIIB(A) 0.1mg/kg
F-T1-SP10IIIB(A) 0.5mg/kg
F-T1-SP10IIIB(A) 0.016mg/kg
T1-SP10IIIB(A) 0.1mg/kg
T1-SP10MN(A) 0.1mg/kg
Δ cpm x 10-5/106
Mononuclear Cells
Month
FIG. 13D
1070
F-T1-SP10IIIB(A) 0.1mg/kg
F-T1-SP10IIIB(A) 0.5mg/kg
F-T1-SP10IIIB(A) 0.016mg/kg
T1-SP10IIIB(A) 0.1mg/kg
T1-SP10MN(A) 0.1mg/kg
Δ cpm x 10-5/106
Mononuclear Cells
Month

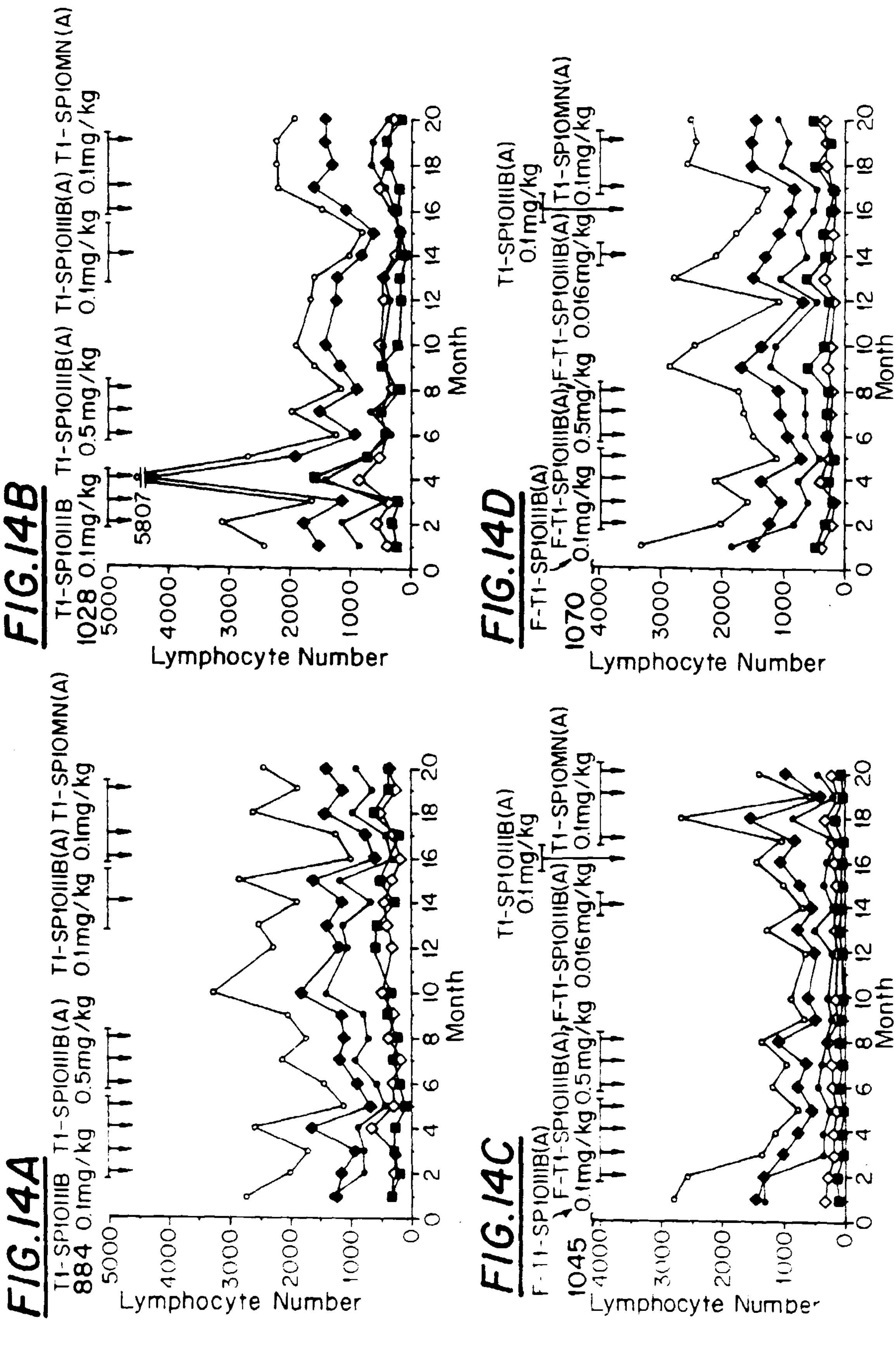
FIG.14A
884
T1-SP10IIIB 0.1mg/kg
T1-SP10IIIB(A) 0.5mg/kg
T1-SP10IIIB(A) 0.1mg/kg
T1-SP10MN(A) 0.1mg/kg
Lymphocyte Number
Month
FIG.14B
1028
T1-SP10IIIB 0.1mg/kg
T1-SP10IIIB(A) 0.5mg/kg
T1-SP10IIIB(A) 0.1mg/kg
T1-SP10MN(A) 0.1mg/kg
5807
Lymphocyte Number
Month
FIG.14C
1045
F-T1-SP10IIIB(A)
F-T1-SP10IIIB(A) 0.1mg/kg
F-T1-SP10IIIB(A) 0.5mg/kg
F-T1-SP10IIIB(A) 0.016mg/kg
T1-SP10IIIB(A) 0.1mg/kg
T1-SP10MN(A) 0.1mg/kg
Lymphocyte Number
Month
FIG.14D
1070
F-T1-SP10IIIB(A)
F-T1-SP10IIIB(A) 0.1mg/kg
F-T1-SP10IIIB(A) 0.5mg/kg
F-T1-SP10IIIB(A) 0.016mg/kg
T1-SP10IIIB(A) 0.1mg/kg
T1-SP10MN(A) 0.1mg/kg
Lymphocyte Number
Month

FIG. 15

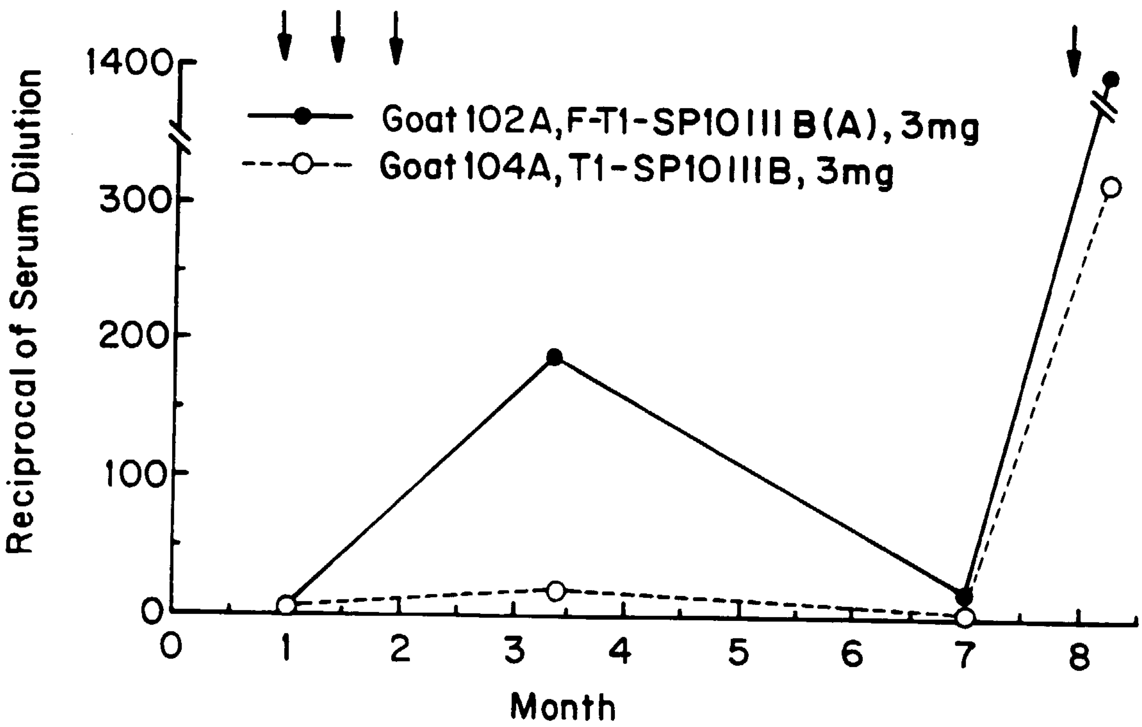
1400
300
200
100
0
Reciprocal of Serum Dilution
Goat 102A, F-T1-SP10111 B(A), 3mg
Goat 104A, T1-SP10111B, 3mg
0
1
2
3
4
5
6
7
8
Month